# FASCICULE DE BREVET EUROPEEN

(11) **EP 0 882 233 B1**
(45) Date de publication et mention de la délivrance du brevet: **26.02.2003**
(21) Numéro de dépôt: 97944930.3
(22) Date de dépôt: 06.10.1997
(51) Int. Cl.: G01N 33/543

(54) **PROCEDE ET DISPOSITIF DE TRAITEMENT PAR COMPLEXATION D'UN MILIEU LIQUIDE**
VERFAHREN UND VORRICHTUNG ZUR BEHANDLUNG DURCH KOMPLEXIERUNG EINES WÄSSRIGEN MILIEUS
METHOD AND DEVICE FOR TREATMENT BY COMPLEXING OF A LIQUID MEDIUM

(30) Priorité: 04.10.1996 FR 9612331
(43) Date de publication de la demande: 09.12.1998
(73) Titulaire: BIO MERIEUX, 69280 Marcy l'Etoile (FR)
(72) Inventeur: GINOT, Frédéric, F-38340 Voreppe (FR)
(74) Mandataire: Guerre, Dominique
(86) Numéro de dépôt international: FR9701781
(87) Numéro de publication internationale: WO98015832

(56) Documents cités:
- EP-A- 0 535 242
- WO-A-92/10588
- WO-A-92/16655
- A. SCHWIENHORST ET AL.: "Hamming chromatography." MOLECULAR DIVERSITY, vol. 1, 1995, pages 187-192, XP000677595
- M. J. DOKTYCZ ET AL.: "Optical melting of 128 octamer DNA duplexes." JOURNAL OF BIOLOGICAL CHEMISTRY, vol. 270, no. 15, 1995, pages 8439-8445, XP002034049 cité dans la demande
- K. J. BRESLAUER ET AL.: "Predicting DNA duplex stability from the base sequence." PROC. NATL. ACAD. SCI., USA, vol. 83, juin 1996, pages 3746-3750, XP002034050 cité dans la demande

## Description

La présente invention concerne de manière générale le traitement par complexation d'un milieu liquide, comprenant un ou plusieurs composants déterminant la présence dans ledit milieu liquide d'une pluralité ou multiplicité de ligands différents à l'état libre.

Dans la présente description, et les revendications, on adopte ci-après les définitions suivantes.

Par "ligand", on entend toute entité ou molécule, de nature chimique, biochimique ou biologique, pouvant se lier de manière spécifique à une autre entité ou molécule, appelée "anti-ligand", par une ou des liaisons non covalentes.

Ressortent en particulier de cette définition différentes entités biologiques ou biochimiques, notamment biopolymères, pouvant former entre elles et de manière complémentaire un complexe :
- il s'agit par exemple d'un polypeptide, en particulier d'un anticorps (ligand) pouvant se lier spécifiquement à un épitope ou antigène (anti-ligand), ou inversement
- il s'agit aussi, comme montré à titre d'exemple dans la description ci-après, de tout acide nucléique ou matériel nucléique, susceptible sous forme monocaténaire de se lier complémentairement avec un oligonucléotide ou un polynucléotide.

La réaction ou équilibre de nature chimique, par lequel le ligand se lie, ou s'apparie, ou se complexe avec l'anti-ligand, sera désignée ci-après par le terme "complexation". Le produit de cette réaction ou équilibre sera désigné ci-après sous le terme de "complexe". S'agissant de matériel ou d'une macromolécule nucléique, le complexe pourra être désigné ci-après sous le terme de "duplex".

Par "matériel nucléique" ou "acide nucléique", on entend toute macromolécule comprenant au moins un enchaînement linéaire, non ramifié, de nucléotides, eux-mêmes modifiés ou non. Ressortent de cette définition, bien évidememnt, tout acide désoxyribonucléique, modifié ou non, et tout acide ribonucléique, modifié ou non. Une telle macromolécule peut être monocaténaire, ou bicaténaire, ou encore avoir tout autre conformation secondaire ou tertiaire. En pratique, dans la description ci-après et à titre d'exemple, ce matériel nucléique sera celui soumis directement au traitement, ou encore celui à partir duquel un milieu liquide est obtenu, contenant le ou les ligands, pour être ensuite traité conformément à la présente invention.

Par "oligonucléotide", on entend tout polynucléotide, comportant au moins cinq monomères, et de préférence au moins sept, par exemple huit monomères, constitués par acide nucléique naturel, ou encore un acide nucléique modifié au niveau d'au moins l'un de ses éléments constitutifs, à savoir du sucre, de la base azotée, ou encore du groupement phosphate.

Par "support", on entend tout substrat à la surface duquel peuvent être immobilisés ou attachés, de manière définitive, directement ou indirectement, des anti-ligands, et ceci aussi bien de manière covalente que non covalente.

Le substrat est ou auto-porteur, par exemple une lame d'un matériau inerte et transparent, ou est lui-même supporté par une base rigide, par exemple une couche d'acrylamide sur une lame de verre.

Par "surface", on entend la surface du substrat accessible aux anti-ligands, qu'il s'agisse d'un substrat plein ou d'un substrat poreux, auquel cas la surface réelle développée est plus importante que la surface apparente.

Dans la description ci-après et à titre d'exemple, les oligonucléotides constitueront les anti-ligands définis précédemment.

La réaction de complexation entre un matériel ou acide nucléique d'une part, et un oligonucléotide complémentaire d'autre part, pourra être désignée ci-après sous le terme d'"hybridation".

Conformément au document WO-92/10558 et WO-93/22680, et selon un format de bio-essai dit "dot blot inverse", on a décrit un procédé et un dispositif de traitement par complexation, à savoir d'hybridation d'un milieu liquide, en l'occurrence d'un échantillon nucléique, comprenant un ou plusieurs composants nucléiques, déterminant la présence dans ce milieu liquide d'une pluralité de fragments, segments ou séquences nucléiques (ligands), différents, à l'état libre. La principale application de ce traitement est le séquençage par hybridation de l'échantillon nucléique, selon des principes et méthode connus exposés dans les documents (6) et (7).

Selon ce procédé :
a) on dispose d'un support de complexation plurale, du type biopuce, sur lequel sont distribués une pluralité de sites ou zones discrètes de couplage, séparés les uns des autres, dans lesquels sont immobilisés une pluralité d'oligonucléotides (anti-ligands) respectivement différents, et susceptibles d'être compléxés avec la ou les séquences nucléotidiques complémentaires du ou des fragments nucléiques, respectivement;
b) dans des conditions isothermes d'un site de couplage à l'autre, on met en contact le milieu liquide, ou échantillon nucléique, avec ce support de complexation, moyennant quoi les séquences nucléotidiques libres s'apparient avec les oligonucléotides fixes respectivement, et sont de ce fait fixées au support de complexation plurale;
c) on observe aux différents sites de couplage du support de complexation plurale une grandeur représentative de la présence et/ou de la quantité des différents complexes ou hybrides obtenus, pour générer des signaux et/ou des informations représentatifs de la présence et/ou de la quantité des différentes séquences nucléotidiques dans le milieu liquide de départ ; préférentiellement ces complexes sont observés en mettant en oeuvre un marquage du ou des composants de l'échantillon nucléique, ou un marquage des différents hybrides obtenus et fixés sur le support.

Comme dit précédemment, ce procédé travaille dans des conditions complètement isothermes, c'est-à-dire en disposant de la même température d'hybridation d'un site de couplage à l'autre, au moment de la mise en contact du milieu liquide avec le support de complexation plurale, et/ou au moment du lavage de ce support après hybridation.

Conformément au document EP-0535242, on a décrit et proposé un procédé similaire, travaillant également dans des conditions isothermes, et selon lequel en chaque site de couplage la concentration en oligonucléotide correspondant et spécifique audit site est choisie en fonction de la température de travail, pour favoriser la stabilité du complexe correspondant obtenu avec la séquence nucléotidique complémentaire.

Selon ce document, on choisit cette concentration en observant les pourcentages de séquence nucléotidique complémentaire éluée en chaque site de couplage, pendant une phase de lavage, effectuée avec un liquide de lavage dont on fait varier la température, et improprement qualifiée de lavage avec gradient thermique.

Les solutions décrites précédemment se heurtent à une difficulté technique, identifiée dans le document WO-92/10588 mais non résolue. En effet, à une température prédéterminée d'hybridation, et pour une longueur de séquence donnée de l'oligonucléotide (anti-ligand), la stabilité du complexe obtenu par hybridation d'un acide nucléique sonde avec sa séquence complémentaire cible varie selon cette séquence. Ainsi deux oligonucléotides ne différant l'un de l'autre que par l'un des nucléotides, à savoir T dans un cas et G et dans l'autre cas, bien que de même longueur et de séquence proche, n'hybrident pas leurs acides nucléiques complémentaires respectifs avec la même stabilité. En d'autres termes, puisque la stabilité des complexes obtenus dépend de la température, il faut hybrider l'acide nucléique échantillon avec les oligonucléotides sondes, portés sur le support de complexation plurale, à une température suffisamment froide pour que le moins stable des complexes possibles puisse se former, sous peine d'obtenir des faux négatifs après hybridation, c'est-à-dire des oligonucléotides sondes ne générant pas de signal d'hybridation, alors que leur séquence complémentaire est présente dans l'échantillon. Cependant, en procédant ainsi, il arrive que des oligonucléotides sondes forment des complexes suffisamment stables avec des séquences non parfaitement complémentaires ; en pareil cas, on obtient alors des faux positifs, c'est-à-dire des oligonucléotides sondes générant un signal d'hybridation, alors que leurs séquences complémentaires ne sont pas présentes dans l'échantillon nucléique.

Comme ces faux négatifs et ces faux positifs introduisent en conséquence des erreurs dans l'analyse de l'échantillon nucléique, notamment pour un séquençage par hybridation, ils rendent la solution technique décrite précédemment souvent inexploitable.

La présente invention a donc pour objet d'augmenter la fiabilité d'un traitement par complexation, notamment hybridation, tel que décrit précédemment.

La présente invention part de la constatation selon laquelle la stabilité ou fidélité de chaque complexe ligand/anti-ligand dépend, directement ou indirectement, d'au moins un paramètre exogène, dit ci-après de référence, et qui conditionne le support de complexation plurale.

Par "paramètre exogène", on entend un paramètre ou une condition extrinsèque par rapport au ligand et/ou à l'anti-ligand, c'est-à-dire indépendant d'eux-mêmes.

Un tel paramètre exogène de référence, conditionnant ou susceptible de conditionner le support de complexation plurale, est par exemple :
- un paramètre ou condition physique, applicable au support, par exemple la température,
- ou un paramètre ou condition chimique, biochimique ou biologique, existant ou disponible à la surface dudit support, par exemple la concentration surfacique d'un anti-anti-ligand, susceptible de s'apparier avec pratiquement tous les anti-ligands,
- ou encore un paramètre physico-chimique, établi sur la surface du support, par exemple sa mouillabilité ou sa tension superficielle par rapport au milieu liquide.

A titre d'exemple encore, par paramètre exogène, s'agissant d'un ligand du type acide nucléique, et d'un anti-ligand du type oligonucléotide, on entend tout paramètre ou caractéristique, n'appartenant pas aux séquences de nucléotides de l'acide nucléique et/ou de l'oligonucléotide, et par exemple différant de leurs richesses respectives en l'une quelconque des bases élémentaires azotées nucléiques.

La présente invention se distingue ensuite par la conjonction des deux caractéristiques suivantes :
- d'une part, dans tout le support, par exemple sur toute sa surface, on différencie les sites de couplage, en fonction du paramètre exogène de référence, lequel a des valeurs respectivement différentes dans les différents sites de couplage
- et on distribue les anti-ligands, entre les différents sites de couplage, en fonction de valeurs optimum prédéterminées du paramètre exogène de référence, déterminant notamment la stabilité des différents complexes ligands/anti-ligands respectivement.

Par conséquent selon la présente invention, chaque site de couplage, sur le support de complexation plurale, auquel est immobilisé un anti-ligand correspondant, travaille à une valeur optimale du paramètre exogène de référence, vis-à-vis de la stabilité du complexe ligand/anti-ligand correspondant, cette valeur optimale pouvant être différente de la valeur optimale d'au moins un site de couplage immédiatement voisin.

Grâce à l'invention, on obtient un mode de complexation plurale particulièrement fiable, précis et sensible, ayant aussi un bon pouvoir de résolution.

Par "couplage", on entend toute liaison, covalente ou non, permettant d'immmobiliser de manière permanente un anti-ligand à la surface du support.

La présente invention est mise en oeuvre selon différents formats de bio-essais, par exemple par complexation directe ou complexation par compétition.

L'invention est éventuellement mise en oeuvre selon un format "sandwich", selon lequel le ligand est susceptible de s'apparier avec l'anti-ligand fixe, et avec un échantillon à l'état libre dans le milieu liquide.

A titre d'exemples préférentiels, trois modalités pratiques différentes sont retenues :
- selon une première modalité, on effectue la mise en contact du milieu liquide avec le support de complexation, en appliquant une même valeur minimale du paramètre exogène de référence, cette valeur minimale étant suffisante pour complexer pratiquement tous les ligands avec les anti-ligands; puis on lave le support en appliquant aux différents sites de couplage des valeurs respectivement différentes et prédéterminées, du paramètre exogène de référence, et ce pendant la seule étape de lavage,
- selon une deuxième modalité, on effectue cette même mise en contact du support avec le milieu liquide, en appliquant aux différents sites de couplage, des premières valeurs respectivement différentes et prédéterminées du paramètre exogène de référence; puis on lave le support en maintenant les différents sites de couplage, à des secondes valeurs prédéterminées, respectivement différentes, du paramètre exogène de référence; les premières valeurs sont identiques ou différentes des secondes valeurs respectivement du paramètre exogène de référence,
- selon une troisième modalité, on effectue la mise en contact du support avec le milieu liquide, en appliquant aux différents sites de couplage des valeurs respectivement différentes et prédéterminées du paramètre exogène de référence, et ce pendant la seule étape de complexation ou hybridation, puis on lave le support hybridé avec un liquide de lavage à température relativement froide et constante.

Bien entendu dans un mode de détection dit de type homogène, une étape de lavage n'est pas indispensable.

La première modalité définie précédemment suppose qu'en chaque site de couplage, la quantité ou concentration d'anti-ligand est suffisante pour se complexer, d'une part avec tous les ligands réellement complémentaires du ligand considéré, et d'autre part avec tous les ligands susceptibles de s'apparier de manière concurrente ou parasitaire avec ce même anti-ligand.

Préférentiellement, on établit sur le support de complexation plurale une gradation spatiale, notamment à plat, par exemple un gradient du paramètre exogène de référence, selon au moins une direction de référence du support, par exemple sa largeur dans le cas d'un support rectangulaire. Et alors, cette gradation spatiale fixe la position ou l'ordonnancement des différents sites de couplage, avec la distribution des anti-ligands entre les différents sites de couplage respectivement.

A titre de variante ou complément à la présente invention, on différencie les sites de couplage sur les supports de complexation plurale, en fonction d'un autre paramètre exogène de référence, ayant lui aussi des valeurs différentes dans lesdits sites de couplage, et d'autre part on distribue les anti-ligands entre les différents sites de couplage, également en fonction de valeurs optimum prédéterminées de l'autre paramètre exogène de référence, qui détermine aussi la stabilité des différents complexes ligand/anti-ligand respectivement.

Préférentiellement, un autre paramètre exogène duquel dépend la stabilité des différents complexes ligand/anti-ligand, est fixé à une valeur moyenne entre celles dudit autre paramètre, requises pour la stabilité des différents complexes ligand/anti-ligand respectivement. A titre d'exemple, cet autre paramètre exogène est le pH ou la force ionique du milieu liquide mis au contact du support de complexation plurale un paramètre dépendant de la composition du tampon d'hybridation.

Le support de complexation plurale, agencé et conditionné selon l'invention en ce qui concerne ses différents sites de couplage, est mis en oeuvre de manière préférée pour la détection et/ou quantification des ligands.

A cette fin, plusieurs modalités sont considérées:
- on observe, notamment par balayage, aux différents sites de couplage du support une grandeur représentative de la présence et/ou de la quantité des différents complexes ligand/anti-ligand, pour obtenir des signaux et/ou des informations représentatifs de la présence et/ou de la quantité des différents ligands dans le milieu liquide de départ
- préférentiellement, on observe les différents complexes ligand/anti-ligand, en mettant en oeuvre au moins une quelconque des opérations suivantes, bien connues en elles-mêmes, à savoir marquage du ou des composants du milieu liquide, et marquage des différents complexes ligand/anti-ligand fixés sur le support.

Classiquement, le milieu liquide mis au contact du support est obtenu à partir d'un échantillon, par exemple un échantillon ou un matériel biologique, soumis à analyse et contenant une ou plusieurs entités différentes; et ceci selon deux modalités:
- ou l'échantillon est traité directement selon le procédé défini précédemment, notamment directement par mise en contact directe avec le support de complexation plurale
- ou l'échantillon est soumis à au moins une opération préalable, choisie pour obtenir un ou plusieurs composants dans le milieu liquide traité, identiques à ou dérivés directement des différentes entités respectivement, et représentatives de ces dernières.

Classiquement encore, le support de complexation sur lequel sont fixés les différents ligands est éventuellement soumis à au moins une opération postérieure, telle qu'un lavage, choisie pour séparer dudit support le ou les composants n'ayant pas été complexés, ou ceux complexés avec les anti-ligands de manière instable, et/ou éluér ou relarguer les différents ligands, de manière différenciée ou non.

Un domaine privilégié, mais non exclusif, d'application de la présente invention, concerne le traitement et l'analyse de produits ou composés nucléiques. Dans ce cas, le ligand est un acide nucléique qui comprend une séquence nucléotidique cible, libre, notamment sous forme monocaténaire, accessible à une hybridation avec une autre séquence nucléotidique sonde, complémentaire, fixe ou fixée, comprise par un anti-ligand correspondant.

Toujours pour un matériel nucléique, à titre préférentiel, le milieu liquide comprend un seul composant, à savoir un acide nucléique, comprenant ou exhihant plusieurs ligands respectivement différents, à savoir plusieurs régions ou segments d'hybridation, moyennant quoi le même composant est apparié aux différents anti-ligands, à savoir oligonucléotides respectivement. Par conséquent, dans ce cas, la suite de nucléotides d'au moins un brin de l'acide nucléique détermine une pluralité de régions d'hybridation, et donc de ligands, selon les séquences nucléotidiques cible, respectivement complémentaires aux séquences nucléotidiques sonde des anti-ligands.

Les traitements selon l'invention appliqués à des produits ou composés nucléotidiques ou nucléiques, sont mis en oeuvre par exemple dans au moins une quelconque des applications suivantes, à savoir séquençage par hybridation du composant nucléique du milieu liquide, reséquençage de ce même composant nucléique, cartographie d'un matériel nucléique, analyse et identification d'un matériel ou échantillon nucléique, typage d'un matériel nucléique, criblage d'un matériel nucléique, analyse génétique (cf par exemple référence bibliographique 8), détection d'un agent pathogène bactérien ou viral.

Une application de l'invention est le séquençage d'un matériel nucléique par hybridation, en particulier en déterminant les séquences chevauchantes de ce matériel. Les principes de ce sêquençage et ses modalités ont été décrites dans les publications (6) et (7).

L'analyse et le traitement des informations ainsi obtenues pour reconstruire la séquence nucléotidique d'un matériel nucléique a été décrit dans les publications (9) et (10).

Une autre application de l'invention est l'identification de microorganismes ou de gènes associés à un microorganisme, par détermination d'un profil d'hybridation, sans reconstitution obligatoire de séquences.

Pour toutes ces applications, le milieu liquide est obtenu à partir d'un échantillon nucléique, comprenant un seul et même fragment nucléique, ou plusieurs fragments nucléiques différents. Et l'échantillon nucléique est éventuellement soumis à au moins une opération préalable, choisie parmi les opérations suivantes, à savoir dénaturation, lyse, fragmentation, clonage, et amplification.

Deux cas différents de traitement selon l'invention doivent être considérés:
- dans un premier cas, le milieu liquide comprend plusieurs composants différents, comprenant des ligands respectivement différents, moyennant quoi lesdits composants différents sont fixés et séparés les uns des autres, et en quelque sorte résolus sur le support de complexation plurale; cette modalité est mise en oeuvre dans au moins une quelconque des applications suivantes, à savoir identification, quantification, séparation et fractionnement des composants différents,
- dans un autre cas, le milieu liquide comprend un seul composant, comprenant ou exhibant lui-même plusieurs ligands respectivement différents, moyennant quoi le même composant est apparié aux différents ligands respectivement.

Un dispositif de traitement conforme à l'invention comprend :
- un support de complexation plurale, sur lequel sont rangés ou distribués, par exemple sous forme matricielle, une pluralité de sites de couplage, séparés les uns des autres, dans lesquels sont immobilisés une pluralité d'anti-ligands respectivement différents, et susceptibles d'être complexés avec lesdits ligands respectivement,
- un moyen de mise en contact du milieu liquide avec le support, agencé pour apparier lesdits ligands avec lesdits anti-ligands respectivement, et les fixer audit support.

Ce dispositif se caractérise en ce que, d'une part il comprend en outre des moyens de différenciation des sites de couplage sur le support, en fonction du paramètre exogène de référence, avec des valeurs respectivement différentes dudit paramètre dans lesdits sites, et d'autre part les anti-ligands sont distribués sur le support, entre les différents sites de couplage, en fonction de valeurs optimum prédéterminées du paramètre exogène de référence, déterminant la stabilité des différents complexes ligand/anti-ligand respectivement.

Préférentiellement, mais non exclusivement, le paramètre exogène de référence retenu étant la température, les moyens de différenciation sont agencés pour graduer la température selon au moins une direction de référence, par exemple selon un gradient de température. Dans ce cas, préférentiellement, le dispositif comprend une source froide et une source chaude, entre lesquelles s'étend ledit support selon la direction de référence.

A titre d'exemple, un tel dispositif comprend un seul ou des capteurs, associés aux différents sites de couplage du support respectivement, détectant au moins une grandeur observée, représentative de la présence et/ou de la quantité du complexe ligand/anti-ligand, en chaque site de couplage, et délivrant un ou des signaux de mesure ou informations correspondantes.

A titre d'exemple, le dispositif comprend un capteur unique, composé lui-même de multiples capteurs élémentaires, par exemple une caméra CDD, ou un capteur unique balayant la surface du support de complexation plurale.

Par exemple, pour l'analyse ou le séquençage d'un matériel nucléique, ce même dispositif comprend ou est associé à une unité de traitement des signaux délivrés par les différents capteurs, selon des opérations mathématiques et/ou logiques prédéterminées, pour obtenir une ou des informations caractérisant le ou les composants du milieu liquide.

A titre d'exemple, la grandeur observée, et représentative de la présence et/ou de la quantité du complexe ligand/anti-ligand, est l'absorbance lumineuse, ou l'intensité d'une lumière émise ou réémise (fluorescence, luminescence, par exemple), l'intensité d'un rayonnement ionisant, ou toute propriété physique, optique, électrique ou diélectrique ou électrochimique des différents complexes ligand/anti-ligand, par exemple diffraction lumineuse, résonnance magnétique nucléaire, variation d'angle de contact, conductivité électrique, voltamétrie, ampérométrie, mesure d'impédance.

A titre de mode de réalisation, le support de complexation plurale comprend l'un des matériaux inertes suivants, du verre, un matériau vitreux, un matériau poreux, inorganique ou amorphe, et une matière plastique.

Lorsqu'il s'agit d'une biopuce, ce support et les sites de couplage sont obtenus selon les techniques traditionnelles de photolithographie (cf publication N°11).

La présente invention est maintenant décrite à titre d'exemple, par référence au traitement d'un acide ou matériel nucléique, selon le protocole expérimental détaillé ci-après, et se référant au dessin annexé dans lequel:
- les Figures 1a à 1d représentent le résultat d'hybridations isothermes respectivement à 4°C, 10°C, 20°C et 25°C, pour des oligonucléotides et fragments nucléiques identifiés de manière abrégée en abscisses ; en ordonnées sont portées les valeurs relatives de la grandeur observée, à savoir de la lumière réémise par fluorescence des différents complexes obtenus; les traits pleins représentent des appariements parfaits, et les traits non pleins représentent des mésappariements externes;
- la Figure 2 représente de manière schématique une vue en coupe d'un dispositif de traitement conforme à l'invention;
- la Figure 3 représente de manière schématique une vue du dessus du dispositif représenté à la Figure 2;
- la Figure 4 représente une vue à échelle agrandie de la cavité de traitement du dispositif représenté aux Figures 2 et 3;
- la Figure 5 représente une vue de dessus du dispositif représenté aux Figures 2 et 3, limitée à la cavité de traitement et de mesure, instrumentée;
- la Figure 6 représente la variation de la grandeur observée (fluorescence) en fonction de la température, pour différents complexes fragment nucléique/oligonucléotide, identifiés dans la légende disposée à droite de cette figure;
- la Figure 7 représente pour une plaque de support de complexation plurale, adaptée à la cavité du dispositif selon Figures 2 et 3, et plus particulièrement représentée à la Figure 4, la disposition selon la largeur ou hauteur de cette plaque, mais aussi selon sa longueur, des sites de couplage, en fonction des oligonucléotides choisis;
- de manière analogue aux Figures 1a à 1d, la Figure 8 représente, avec les mêmes conventions que dans les Figures précitées, l'hybridation obtenue avec les fragments nucléiques et oligonucléotides identifiés en abscisses, et ceci avec un support de complexation plurale selon l'invention.

### PROTOCOLE EXPERIMENTAL

Selon le plan suivant:
**I - Mise en évidence du problème à la base de la présente invention**
   1.1 Choix des oligonucléotides (anti-ligands)
   1.2 Synthèse des oligonucléotides
   1.3 Méthode de greffage des oligonucléotides
   1.4 Hybridations et visualisation de la fluorescence des complexes
   1.5 Résultats d'hybridation à températures isothermes
**II - Résolution du problème à l'aide d'un support de complexation plurale, dont les sites de couplage sont différenciés selon un gradient thermique**
   2.1 Description du dispositif selon l'invention permettant l'application d'un gradient thermique
   2.2 Positionnement des oligonucléotides à leur température optimum prédéterminée d'hybridation
   2.3 Résultats d'hybridation en gradient thermique, selon l'invention
   2.4 Extrapolation au fonctionnement d'une "biopuce" à oligonucléotides universelle

### I - Mise en évidence du problème à la base de la présente invention

### 1.1 - Choix des oligonucléotides (anti-ligands)

Pour montrer les difficultés rencontrées pour une hybridation traditionnelle d'acides nucléiques (ligands) avec des oligonucléotides immobilisés à la surface d'un support solide, des oligonucléotides modèles représentatifs dans ce type d'analyse ont été choisis.

La longueur des oligonucléotides choisis est de 8 bases: c'est une longueur communément admise pour une application de type séquençage par hybridation. (références bibliographiques 1 et 2).

3 séquences de base , notées B, D, et J, ont été choisies. Elles se caractérisent par les différences de stabilité des ADNs double brin correspondant. La séquence B, riche en nucléotides A et T, formera un duplex relativement instable avec sa séquence complémentaire. En revanche, la séquence J, riche en nucléotides C et G, formera un duplex relativement stable avec sa séquence complémentaire. La séquence D, contenant autant de nucléotides A et T que de nucléotides C et G formera un duplex de stabilité intermédiaire avec sa séquence complémentaire. Il est possible de prédire approximativement les stabilités relatives d'oligodeoxyribonucléotides double-brins en utilisant le modèle du voisin le plus proche de Breslauer et col. (cf référence bibliographique 3). Ce modèle permet de calculer pour chaque duplex et à toute température l'énergie libre de liaison en fonction de sa séquence.

A partir de ces trois séquences de base (notées B, D, et J, voir ci-après), sept autres séquences ont été déterminées, qui correspondent à des variations simples des séquences B, D ou J.

Ce sont ces 10 oligonucléotides qui ont été immobilisés sur un support solide de complexation plurale. Ils contiennent un groupe biotine à leur extrémité 5', nécessaire à leur immobilisation (voir §1.3 ci-après). Dans le tableau ci-dessous les trois séquences de base sont écrites en gras. Dans la rubrique "remarque", le suffixe "c" désigne la séquence complémentaire.

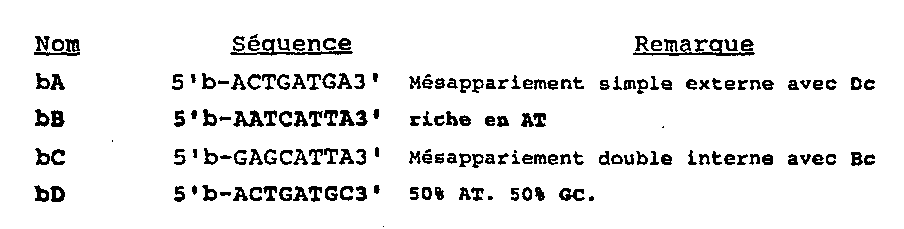

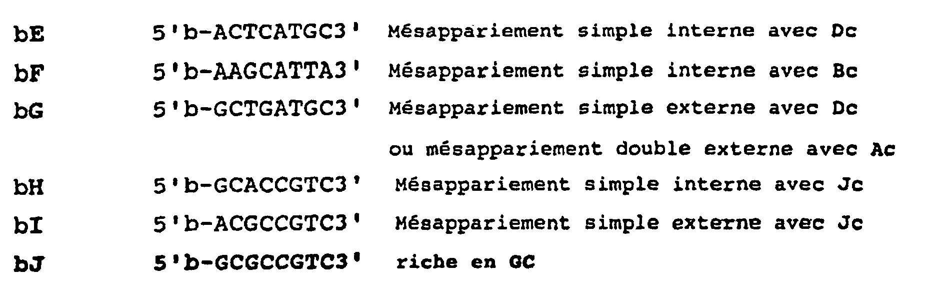

6 oligonucléotides fluorescents (ligands marqués) ont été hybridés avec ces oligonucléotides immobilisés. Il s'agit des oligonucléotides complémentaires des 3 séquences de base, et de leurs variants différant par un nucléotide terminal. Ils contiennent une fluorescéine à leur extrémité 5'.

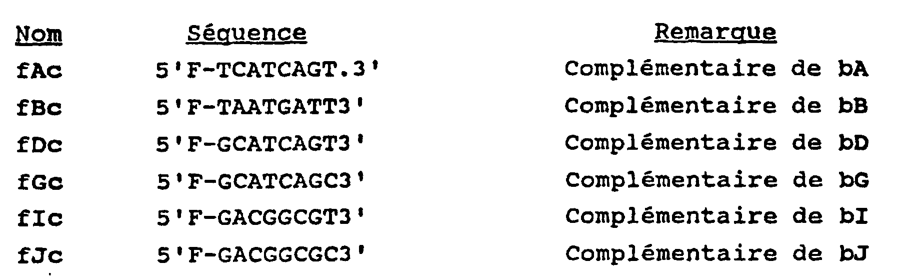

### 1.2 - Synthèse des oligonucléotides

Les oligonucléotides marqués par la biotine et la fluoresceine ont été obtenus après couplage des dérivés activés de biotine et de fluoresceine avec les séquences oligonucléotidiques fonctionnalisées par un bras aminé -(CH₂)₆-NH₂- en position 5'.

### 1.2.1 - Synthèse des oligonucléotides

Les oligonucléotides aminés ont été synthétisés sur un appareil ABI 394 (Applied Biosystems, Forster City, CA) selon la méthode au phosphoramidite en utilisant le protocole donné par le constructeur. Tous les réactifs, y compris le précurseur phosphoramidite du bras aminé, sont fournis par Applied Biosystems. Après clivage du support et déprotection, les séquences oligonucléotidiques sont précitées par addition d'une solution d'acétate de sodium (3M) et de l'éthanol froid (-20°C).

### 1.2.2 - Synthèse du conjugué oligonucléotidebiotine en 5'

L'oligonucléotide aminé est séché puis solubilisé dans une solution tampon de carbonate de sodium 0,2M, NaCl 0,15M (pH 8,8). Une solution de biotine (biotinoylamidocaproic acid N-hydroxysuccinimide ester, BOEHRINGER) dans la DMF (5mg/l) est ensuite ajoutée. Après 2 heures d'incubation à 37°C, la réaction est bloquée par addition de 10 microlitres d'une solution de chlorure d'ammonium (1M). L'oligonucléotide biotinylé est ensuite précipité à -80°C dans de l'éthanol et de l'acétate de sodium (3M), solubilisé dans de l'eau pure puis purifié par HPLC en phase inverse. Sa concentration a été déterminée par mesure UV à 260 nm.

### 1.2.3 - Synthèse du conjugué oligonucléotidefluoresceine en 5'

L'oligonucléotide aminé est séché puis solubilisé dans une solution tampon de carbonate de sodium 0,2M, NaCl 0,15M (pH8,8). Une solution de l'isothiocyanate de fluoresceine (ALDRICH) dans la DMF (12,5mg/l) est ensuite ajoutée. Après 2 heures d'incubation à 55°C, la réaction est bloquée par addition de 25 microlitres d'une solution de chlorure d'ammonium (1M). L'oligonucléotide fluorescent est ensuite précipité et purifié comme indiqué ci-dessus. Sa concentration a été déterminée par mesure UV à 260nm.

La pureté des différents conjugués est contrôlée par HPLC en phase inverse.

### 1.3 - Méthode de greffage (immobilisation) des oligonucléotides (anti-ligands)

Le support solide sélectionné pour les expériences est une lame de microscope en verre. C'est un support courant pour ce genre d'analyse. Les lames utilisées sont de dimension 75 x 25 x 1 mm. Le procédé d'immobilisation des oligonucléotides mis en oeuvre est composé de techniques couramment utilisées en dérivation de surface de verre et en biologie moléculaire.

La première étape consiste à greffer des groupes amines à la surface du verre, au moyen d'une silanisation. Dans la seconde étape, la surface est activée au moyen d'un agent de couplage réagissant fortement avec les amines. Dans une troisième étape, de l'avidine est greffée de façon covalente sur la surface activée, par la réaction des amines primaires de ses acides aminés sur l'agent de couplage. Enfin, dans une dernière étape, les oligonuléotides biotinilés sont immobilisés à la surface, grâce à la fixation forte de la biotine par l'avidine.

Ce procédé assure une fixation des oligonucléotides sur la surface de verre par leur extrémité 5'.

### Protocole détaillé :

1. Laver les lames de verre à l'acide sulfochromique (Prolabo) dilué au dixième, les rincer à l'eau, et les faire sécher à 80°C pendant 15 minutes.
2. Plonger les lames dans une solution de toluène contenant 1 % (vol/vol) de 3-aminopropyldimethylethoxysilane (société ABCR). Laisser incuber 20 minutes à température ambiante.
3. Rincer deux fois les lames à l'éthanol, et les faire sécher 15 min à 80°C.
4. Plonger les lames dans du dimethylformamide contenant 10% (vol/vol) de pyridine, et 0,2% (w/vol) de 1,4 phenylène diisothiocyanate (Aldrich).
   Laisser incuber 1 h à température ambiante.
5. Rincer les lames dans du méthanol, puis de l'acétone, et les faire sécher 15 min. à 80°C.
6. Déposer des gouttes de 2µl d'avidine en solution, aux endroits voulus. La solution d'avidine est composée de Tris-HCI 10 mM pH8; EDTA 1 mM; Avidine 1 mg/ml (Sigma). Laisser incuber 20 à 30 minutes à température ambiante.
7. Reprendre les gouttes d'avidine à la pipette.
8. Rincer les dépôts d'avidine avec une solution de NACl 1 M, toujours en déposant des gouttes de 2 µl et en les reprenant à la pipette.
9. Déposer des gouttes de 2 µl d'oligonucléotides biotinilés (5µM) aux mêmes endroits. Laisser incuber 30 minutes à température ambiante. Retirer les gouttes à la pipette. Les oligonucléotides sont dilués en Tris-HCI 10 mM, pH8; EDTA 1 mM; NaCl. 1.M.
10. Plonger les lames dans une solution ammoniaque 1% (vol/vol), NaCl 1 M. Les laisser 10 minutes à température ambiante.
11. Rincer les lames à l'eau, et les faire sécher à l'air ambiant.

Les lames sont alors prêtes pour l'hybridation ou complexation.

Pour déterminer les sites de couplage et positionner les oligonucléotides de façon précise sur les lames de verre, les étapes 6 à 9 ont été automatisées au moyen d'un robot Gilson, modèle 222. Ce robot est composé d'un bras robot cartésien muni d'une aiguille reliée à un diluteur. Ce montage permet le dépôt et la reprise de gouttes de façon précise et reproductible.

### 1.4 - Hybridations et visualisation de la fluorescence (grandeur caractérisant la présence et/ou la quantité de complexe)

Les oligonucléotides fluorescents ont été hybridés sur la surface portant les oligonucléotides immobilisés à une concentration de 10⁻⁷ M dans le tampon suivant: 50 mM Tris-Cl pH 8; 4,5 M TMACl; 2 mM EDTA; 0,01% sarcosine. Ce tampon provient de la référence bibliographique 4.

Le volume d'hybridation est de 150 µl. Ce volume correspond à un film liquide d'environ 0,1 mm d'épaisseur quand des lamelles 24x50 mm sont utilisées.

La durée de l'hybridation est de 1 h.

Les hybridations isothermes ont été faites entre lame et Lamelle, dans une étuve ou un bain-marie assurant la température désirée.

Pour les hybridations selon l'invention avec gradient thermique (voir ci-après), la solution d'hybridation (milieu liquide) est déposée directement sur le bloc de métal supportant le gradient, et la lame portant les oligonucléotides a été appliquée dessus, en prenant soin d'éviter la formation de bulles.

La visualisation ou observation des hybridations (complexations) a été faite par fluorescence, au moyen d'un appareil FluorImager de la société Molecular Dynamics (Sunnyvale, Californie, USA). Cet appareil balaie la surface à observer en l'illuminant point par point, et site de couplage par site de couplage, au moyen d'un faisceau laser argon (excitation à 488 nm), et collecte la lumière réémise, observée et détectée au moyen d'un faisceau de fibres optiques. Un filtre passe-baut à 515 nm est inclus de façon inamovible dans L'appareil. Il est possible d'ajouter des filtres supplémentaires pour mieux sélectionner en longueur d'onde la lumière collectée et observée.

Pour les observations, un filtre d'interférence 530 ± 30 nm a été ajouté, et le photomultiplicateur de l'appareil a été réglé sur 920 Volts. La taille du pixel de l'impage résultat est un carré de 200 x 200 µm. Et un site de couplage consiste en un disque d'environ 2 mm de diamètre, ce qui représente une image finale de chaque site de l'ordre d'une dizaine de pixels.

Les oligonucléotides (anti-ligands) sont de courte taille, et une dissociation rapide des duplex d'ADN a lieu à température ambiante pour les séquences ayant les plus faibles stabilités (séquences A et B). Aussi le procédé suivant a-t-il été mis au point pour observer de façon fidèle les hybridations:
1. Plonger rapidement les lames dans du tampon d'hybridation refroidi à -20°C. Agiter.
2. Plonger rapidement les lames dans du SSC 20X (référence commerciale de la Société AMBESCO, Solon, Ohio, USA) refroidi à -9°C. Agiter.
   Ces deux étapes doivent prendre 10 à 15 secondes maximum.
3. Plonger les lames rapidement dans du tampon d'hybridation refroidi à -20°C. Il n'y a pas de dissociation du duplex (complexe) à cette température et dans ce tampon. Ce tampon doit être dégazé.
4. Sortir les lames de la solution comprenant les oligonucléotides des cibles (ligands). Déposer une lamelle préalablement refroidie dessus. Essuyer rapidement le dessous de la lame.
5. Visualiser et observer aussitôt avec l'appareil Fluorimager.

La quantification des hybridations a été réalisée au moyen du logiciel ImageQuant® fourni par la Société Molecular Dynamics avec l'apppareil FluorImager.

Les protocoles décrits ci-dessus assurent un coefficient de variation des signaux d'hybridation de 25% pour des duplicata portés sur la même lame.

### 1.5 - Résultats d'hybridation à température isotherme

Des lames portant les 10 oligonucléotides (anti-ligands) bA, bB, bC, bD, bE, bF, bG, bH, bI, bJ ont été produites, et ont été hybridées à 4°C avec un mélange des trois oligonucléotides (ligands) fluorescents fBc, fDc, et fJc, chacun à la concentration initiale de 10⁻⁷ M.

Les résultats quantitatifs de ces hybridations sont résumés dans le tableau 1.

Il s'agit de la moyenne d'une expérience, en duplicat, et les signaux d'hybridation ont été exprimés en fonction du signal du duplex bJ:FJc.

A partir des valeurs du tableau 1, la différence d'intensité des signaux d'hybridations fortes et très faibles est d'environ 15 fois. Une hybridation non délectable est au moins 20 fois plus faible qu'une hybridation forte.

Comme attendu, les hybridations obtenues avec les oligonucléotides bB, bD, et bJ sont fortes, puisque les oligonucléotides libres en solutions étaient leurs complémentaires respectifs.

Les hybridations obtenues avec bC, bE, bF, et bH sont très faibles ou non détectables, ce qui signifie que les mésappariements internes sont facilement distingués des appariements parfaits.

En revanche, il n'en est pas de même pour les mésappariements externes. En effet, si bA génère un signal d'hybridation relativement faible, il n'en est pas de même pour bG et bI qui gênèrent des signaux d'hybridation aussi forts que ceux des appariements parfaits. Le mésappariement externe formé par le duplex bA:fDc est donc correctement discriminé, mais les mésappariements externes que forment les duplex bG:fDc et bI:fJc ne le sont pas.

Dans ces conditions expérimentales, on voit donc qu'il n'est pas possible d'analyser finement un échantillon (milieu liquide) inconnu. Si l'hybridation ci-dessus avait été menée avec un échantillon inconnu, les oligonucléotides bB, bD, bG, bI, et bJ auraient été déterminés comme hybridant avec l'échantillon, ce qui représente un taux de faux positif de 40%.

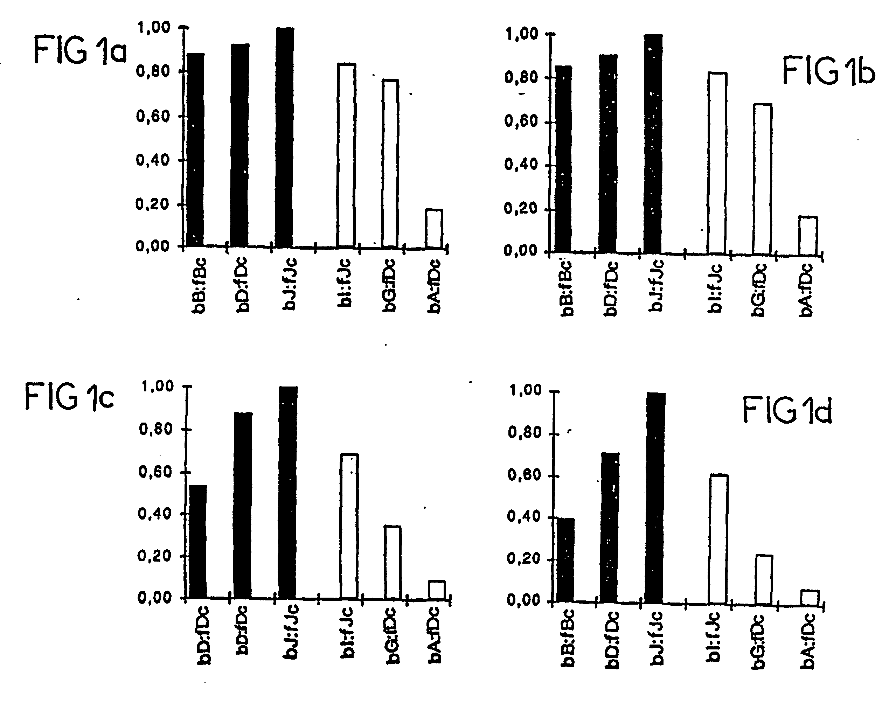

Pour améliorer la discrimination des mésappariements externes, on peut penser à augmenter la température d'hybridation. Pour explorer cette solution, les six oligonucléotides bA, bB. bD, bG, bI et bJ ont été immobilisés sur une lame, et hybridés avec les oligonucléotides fBc, fDc, et fJc à différentes températures (4, 10, 20, 25, 30, et 37°C). La figure 1 représente les résultats d'hybridation obtenus pour les températures de 4 à 25°C. Il s'agit des moyennes obtenues sur 16 à 42 déterminations. Les résultats sont exprimés en intensité lumineuse relative de l'hybridation bJ:fJc, car ce duplex apparait être le plus stable. Bien entendu, l'intensité absolue du signal d'hybridation de ce duplex diminue quand la température augmente.

La figure 1 montre qu'à 10°C le profil des hybridations est semblable à celui obtenu avec des hybridations à 4°C. En revanche, certains changements ont lieu à 20 et 25°C. Le mésappariement externe bG-fDc devient nettement plus faible que l'appariement parfait bI:fJc. De même, le mésappariement externe bI:fJc devient significativement plus faible que l'appariement parfait bJ:fJc, bien que la différence soit un peu faible pour une discrimination parfaite lors d'une hybridation unique. Cependant, l'appariement parfait bB:fBc diminue lui aussi d'intensité, et génère un signal plus faible que celui du duplex mésapparié bI:fJc. En conséquence, il n'est pas possible de choisir un seuil d'intensité d'hybridation qui départage sans erreur les duplex parfaitement appariés des duplex mésappariés. Par exemple, si l'on choisit d'hybrider à 25°C avec un seuil de 0,5, l'oligonucléotide bB sera déclaré négatif, et l'oligonucléotide bI sera déclaré positif. Le résultat final comprendra donc un faux positif et un faux négatif. Si l'on choisit alors une hybridation à 20°C avec un seuil de 0,4, l'oligonucléotide bB sera bien déclaré positif, mais l'oligonucléotide bI également, ainsi que, parfois. l'oligonucléotide bG, selon les variations expérimentales.

### II - Solution selon l'invention avec un gradient thermique

### 2.1 - Description du montage permettant l'application d'un gradient thermique

Pour résoudre le problème de discrimination des mésappariements externes exposé ci-dessus, on a conçu et développé un dispositif de traitement, capable d'établir un gradient thermique à la surface de la lame de verre portant les oligonucléotides immobilisés, et constituant un support de complexation plurale.

Ce dispositif répond aux spécifications suivantes:
- gradient de température de I0°C dans la zone utile ou de travail longue de 1 cm
- connaissance de la température du support de la lame en tout point à 0,1°C près
- reproductiblité des températures du support de lame d'un essai à l'autre : 0,1°C, à 0,1 mm près; la température ambiante doit pouvoir varier librement de 20 à 25°C
- facilité d'installation et d'enlèvement de la lame de verre portant les oligonucléotides.

La façon la plus simple d'établir un gradient thermique à la surface d'un support est de connecter, les extrémités de ce support à une source froide 3 et à, une source chaude 4 respectivement. Il s'établit alors un gradient thermique entre les deux sources. Si le support a une épaisseur constante, le gradient de température est constant, c'est-à-dire que la température varie linéairement sur le support en fonction de la distance le long de ce dernier.

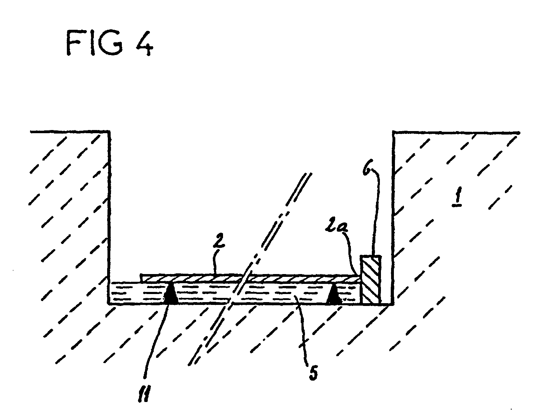

Du point de vue pratique, il est nécessaire que la lame de verre 2 sur laquelle sont immobilisés les oligonucléotides puisse être facilement remplaçable d'un essai à l'autre, et qu'il n'y ait pas de différence d'échanges thermiques entre les différents essais. C'est pourquoi le gradient thermique n'a pas été établi directement dans la lame de verre, mais dans un bloc métallique 1 à la surface duquel une petite cavité 1a a été creusée. La lame de verre 2 est posée dans cette cavité, la face portant les oligonucléotides immobilisés dessous. Entre le bloc métallique 1 et la lame de verre 2 supportée par des cales 11, se trouve la solution 5 d'hybridation (cf Figure 4), ou milieu liquide.

Dans la figure 4 sont indiqués des butées 6 de positionnement de la lame de verre 2. En effet, la lame est placée en contact avec ces butées. Lors de l'immobilisation des oligonucléotides sur la lame, la position des oligonucléotides dans le gradient thermique est déterminée à partir du bord 2a de la lame venant ainsi en butée. Le bloc métallique 1 est en acier inox 3041, ayant par exemple une conductivité thermique entre 10 et 50 W/m.K; il est isolé par une enveloppe 7 ayant une conductivité intrinsèque par exemple inférieure à 0,06 W/m.K, par exemple en polyuréthanne.

Les extrémités du dispositif ont été connectées à des bain-mariés à circulation des sources froide et chaude, dont la régulation thermique est stable à ±0,02°C selon les indications du constructeur (cryothermostat ministat Huber, -25/+120°C).

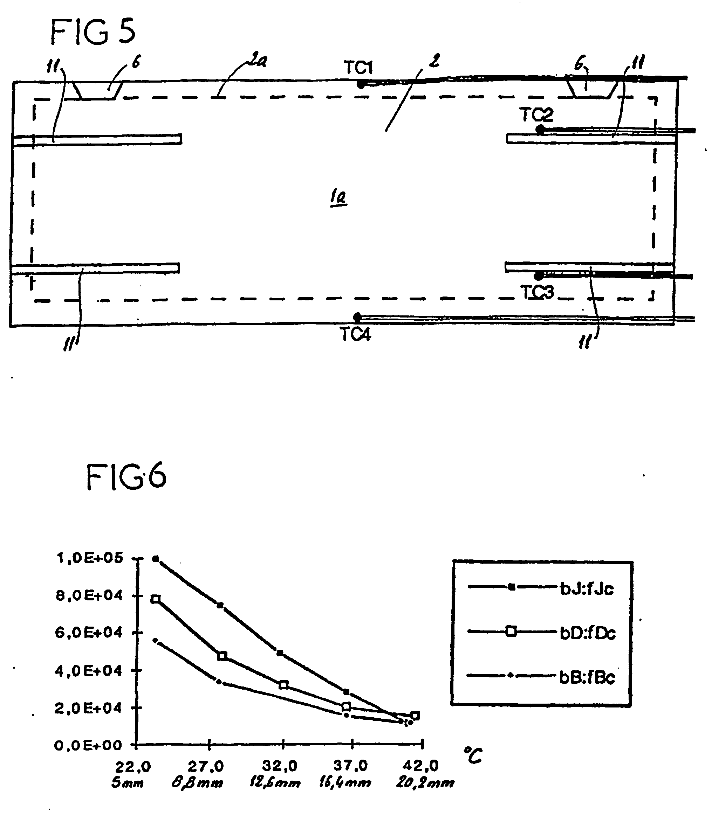

Les températures du dispositif ont été controlées au moyen des thermocouples chromel-alumel (type K) décrits dans la figure 5. Les thermocouples TCI et TC4 sont situés de part et d'autre de la lame 2, alors que les thermocouples TC2 et TC3, dont les fils ont un diamètre total de 0,08 mm, sont situés sous la lame de verre, dans la solution d'hybridation. Ces thermocouples ont été soudés par décharge électrique directement sur l'acier. Leurs positions (médianes entre les deux fils des thermocouples) ont été déterminées à 0,02 mm près, au moyen d'une loupe binoculaire surplombant une table x,y micrométrique. Ils sont montés en différentiel avec une soudure froide plongeant dans un bain d'eau pure glacée. Les tensions différentielles sont mesurées au moyen d'un multimètre de haute précision (Keithley, modèle 2000. Précision : 0,1 µV), et traduites en températures au moyen de la table standard pour ces thermocouples.

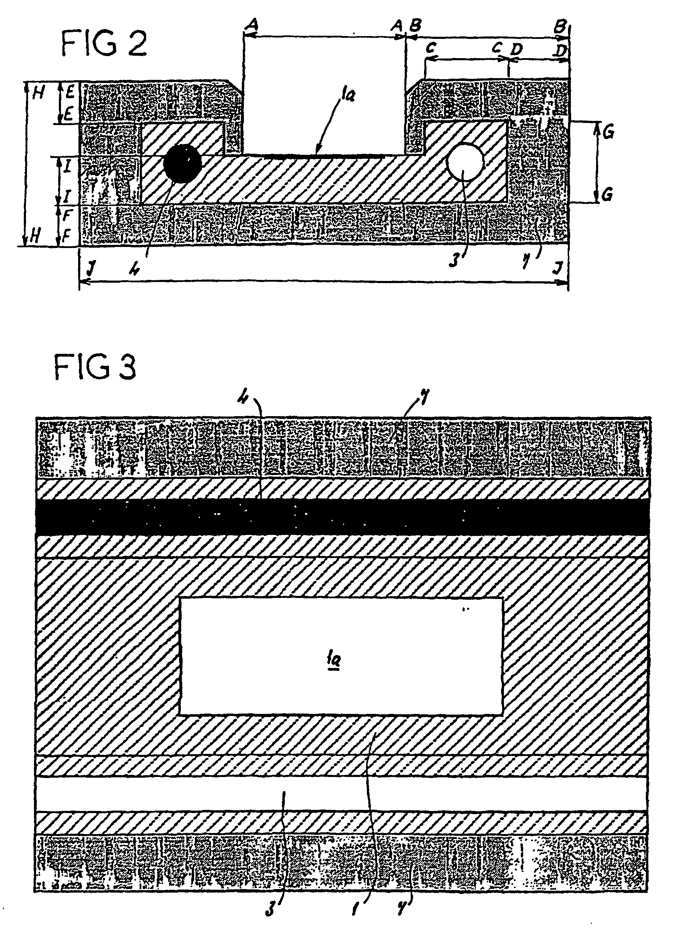

Le dimensionnement du dispositif de traitement précédemment décrit est montré par les repères lettrés de la Figure 2 et est : AA:40mm, BB:40mm, CC:20mm, DD:15mm, JJ:120mm, EE:10mm, FF:10mm, GG:20mm, HH:40mm, II:12mm.

La linéarité du gradient thermique, prédite par les simulations numériques, a été vérifiée expérimentalement au moyen des thermocouples placés dans le montage. Le gradient étant linéaire, les températures en tout point de la zone utile de la lame 2 peuvent être calculées simplement à partir des températures données par les thermocouples TC2 et TC3.

Les mesures de température qui ont été faites pour plusieurs températures des sources chaude et froide, et à différentes reprises, montrent que la répartition des températures est reproductible d'un essai à l'autre, à 0,1°C près.

Dans la plupart des expériences reportées ici, le bain-marié froid était régulé à -9°C, et le bain-marie chaud à +61°C. Le liquide de la source froide 3 était de l'eau glycolée (25% glycol), et celui de la source chaude 2 de l'eau pure. Le gradient établi était de 1,085°C/mm. Les deux bords de la lame de verre, distants de 25 mm, se trouvaient respectivement à 13,2 et à 40,3°C.

### 2.2 - Positionnement des oligonucléotides à leur température optimum prédéterminée d'hybridation

Les résultats des hybridations isothermes montrent que la température d'hybridation de chaque oligonucléotide doit être suffisamment chaude pour qu'il y ait une discrimination correcte des mésappariements externes. Mais cette température doit rester en même temps suffisamment basse pour que le signal d'hybridation soit détectable. Avec le dispositif précédemment décrit, on a fixé la température d'hybridation, de telle façon que le signal d'hybridation soit égal à 25% du signal d'hybridation obtenu à 4°C, qui correspond à la saturation des oligonucléotides immobilisés. On détermine ainsi approximativement le positionnement et la distribution des oligonucléotides dans le gradient thermique, à la surface de la lame de verre 2 (support plan).

Pour affiner les positions respectives des oligonucléotides dans le gradient, des lames ont été produites qui portaient les oligonucléotides en quintuplets dans la direction du gradient. Les 5 sites de couplage d'un quintuplet se trouvent respectivement à 5, 8,8, 12,6, 16,4, et 20,2 mm du bord froid 2a de la lame 2. Ces lames ont ensuite été hybridées avec diverses combinaisons des oligonucléotides fluorescents complémentaires. La figure 6 est le résultat d'une telle hybridation avec les oligonucléotides fBc, fDc, et fJc. Dans cette expérience, les bains-maries du gradient thermique étaient réglés à -9 et +71°C.

A l'issue de quelques expériences de ce type, les localisations des sites de couplage des différents oligonucléotides respectivement ont été définies :
bA : 23,5°C; bB : 23°C; bD : 26,5°C; bG : 27,5°C; bl : 27,5°C; bJ : 29°C

### 2.3 - Résultats d'hybridation en gradient thermique, selon l'invention

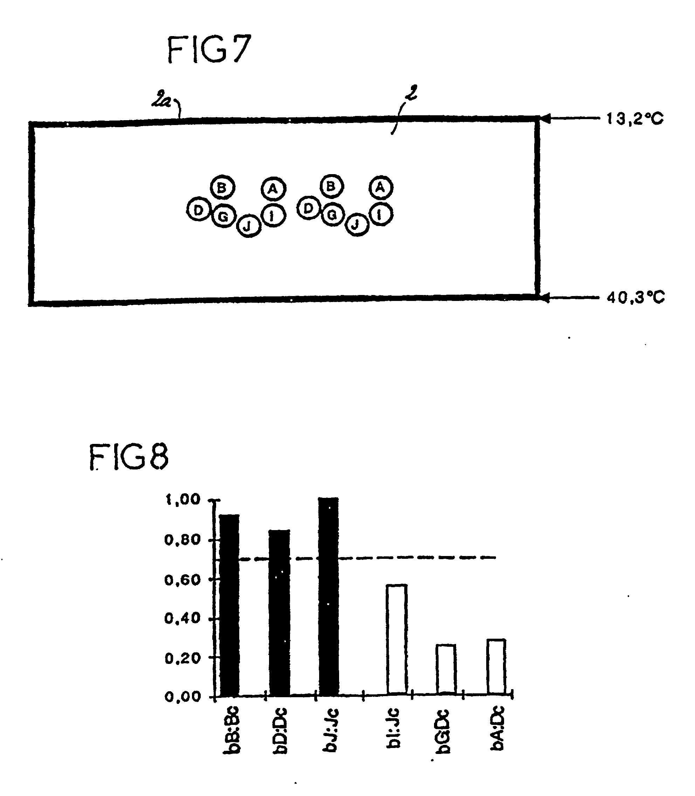

Pour ces expériences, les oligonucléotides bA, bB, bD, bG, bI, et bJ ont été immoblisés sur des lames de verre respectivement à 9,5 , 9 , 12,2 , 13,2 , 13,2 , et 14,6 mm du bord froid 2a de la lame 2. Quand les bains-maries des sources froide et chaude sont réglés sur -9 et +61°C, ces positions correspondent aux températures définies ci-dessus. Les oligonucléotides ont été déposés en duplicat, selon le schéma de la figure 7. Les lames 2 ont ensuite été hybridées en gradient thermique, avec le même mélange des oligonucléotides fBc, fDc, et fJc utilisés précédemment dans les hybridations isothermes.

Les résultats d'hybridation obtenus sont résumés dans la figure 8. Il s'agit des moyennes obtenues sur 5 déterminations. Les résultats sont exprimés en intensité relative de l'hybridation bJ:fJc, comme pour les résultats exposés aux figures la à 1d.

On remarque que les trois appariements parfaits génèrent un signal d'hybridation d'intensité comparable (de 0,84 à 1), alors que les mésappariements externes donnent des signaux significativement plus faibles. Contrairement aux résultats obtenus en hybridation isotherme, il est possible ici de définir un seuil d'intensité qui départage les appariements parfaits des mésappariements, par exemple un seuil de 0,7.

Afin de vérifier le bon fonctionnement du dispositif précédemment décrit, des lames identiques ont été hybridées avec d'autres combinaisons des oligonucléotides fluorescents fAc, fBc, fDc, fGc, fIc, et fJc. Ces combinaisons comprenaient systématiquement l'oligonucléotide fBc. Elles comprenaient en outre fIc ou fJc, et fAc, ou fDc, ou fGc. De cette façon, les trois oligonucléotides fluorescents de chaque combinaison n'interféraient pas dans leurs hybridations car ils ne s'appariaient jamais sur un même oligonucléotide fixe. Une combinaison contenant fAc, fGc, et fIc a aussi été utilisée. En effet, les hybridations croisées entre les séquences A et G sont très faibles (voir résultats ci-dessous).

Les résultats de ces hybridations sont rapportés dans le tableau 2. Il ont été exprimés relativement à l'intensité du signal d'hybridation du duplex bB:fBc. De plus, pour les mésappariements, les résultats ont aussi été exprimés relativement à l'intensité du signal d'hybridation de l'appariement parfait correspondant.

Ces résultats montrent clairement que tous les appariements parfaits génèrent des signaux d'intensité semblable. Cependant, l'appariement bG:fGc génère un signal légèrement trop faible. puisqu'il est de 0,79 avec un écart-type sur 6 déterminations de 0,22. La probabilité que le signal réel, en dehors des variations expérimentales, soit effectivement plus petit que 1 est donc forte, d'environ 97%. Ceci signifie donc que le positionnement de G dans le gradient thermique est un peu chaud dans ces expériences, et que G peut être repositionné vers la source froide.

Par ailleurs, ces résultats montrent également que les mésappariements externes génèrent des signaux nettement plus faibles que le duplex parfait bB:fBc, ou encore que leurs homologues parfaits respectifs. Comme pour le cas particulier des hybridations avec la combinaison fBc+fDc+fJc, un seuil de 0,7 par rapport à un duplex parfait de référence permet de distinguer les appariements parfaits des mésappariements externes.

De nouvelles expériences ont alors été faites, avec des lames sur lesquelles l'oligonucléotide bG était positionné à 26,8°C (12,5 mm du bord froid 2a de la lame 2), la position des autres oligonucléotides restant inchangée. Ces lames ont été hybridées avec les combinaisons d'oligonucléotides fluorescents fBc+fGc+fIc et fBc+fGc+fJc. La moyenne du signal d'hybridation du duplex bG:fGc par rapport au duplex bB:fBc alors été de 1,08 sur 7 déterminations, avec un écart-type de 0,32. On a alors extrapolé les signaux des mésappariements bG:fDc et bG:fAc à cette nouvelle température de bG en prenant les anciennes valeurs, et en les multipliant par le rapport 1,08 : 0,79 = 1,367. De même, les signaux des mésappariements bD:fGc et bA:fGc par rapport à leur homologue parfait correspondant bG:fGc ont été déduits des valeurs précédentes en divisant celles-ci par 1,367. L'ensemble des résultats et de ces calculs est donné dans le tableau 3.

Comme attendu, ces nouveaux résultats confirment et améliorent les précédents. Tous les appariements parfaits génèrent un signal d'hybridation proche de 1, alors que les mésappariements terminaux génèrent des signaux inférieurs à 0,5, sauf bI:fJc qui est légèrement supérieur. Un seuil de 0,7 permet de distinguer indiscutablement les appariements parfaits des mésappariements externes.

### 2.4 - Application au fonctionnement d'une biopuce à oligonucléotides universelle

Lors de l'utilisation d'une biopuce à oligonucléotides portant par exemple l'ensemble des 65 536 octamers possibles, il n'est pas très efficace d'appliquer simplement un seuil d'intensité pour déterminer si un signal est positif ou négatif. En effet, les signaux des mésappariements les plus stables étant seulement deux fois plus faibles que les signaux des appariements parfaits, il est probable que des variations expérimentales feront qu'un certain nombre d'appariements parfaits seront détectés comme négatifs, et que de même, un certain nombre de mésappariements externes seront déterminés comme positifs. Par exemple, sur l'ensemble des données résumées dans le tableau 3, il y a 2 signaux d'appariements parfaits sur les 39 reportés, qui sont en dessous du seuil de 0,7 (5.1%), et il y a 2 des 56 (3,6%) déterminations de mésappariements externes qui sont au dessus de ce seuil de 0,7. Il s'agit d'une valeur pour le duplex bJ:fIc, et d'une valeur pour le duplex bJ:fIc. Ces deux duplex font partie des trois duplex mésappariés les plus stables des expériences conduites.

Une façon plus efficace de procéder est d'associer tous les oligonucléotides ne différant que par leurs nucléotides terminaux dans un même groupe (16 octamers pour une biopuce portant tous les octamers possibles), et de déterminer pour chaque groupe le signal le plus intense. Il est alors facile de distinguer les groupes positifs des groupes négatifs, car cela reviendra à distinguer des appariements parfaits et des mésappariements internes. Par exemple, on applique un seuil de 0,4 par rapport aux. signaux les plus intenses.

Ensuite, pour chaque groupe d'oligonucléotides, on peut appliquer le seuil de 0,7 par rapport au signal le plus intense.

Si l'on procède de cette façon sur l'ensemble des expériences rapportées dans le tableau 3, aucun appariement parfait n'est déterminé comme négatif car tous sont largement au dessus du seuil de 0,4 par rapport au signal le plus intense de l'expérience. En revanche, les deux valeurs qui étaient faussement déterminées comme positives restent faussement positives.

Par application de ces données, une biopuce à oligonucléotides universelle fonctionnant de cette façon aurait donc un taux de faux négatif de 0%, un taux de faux positifs des mésappariements internes de 0%, et un taux de faux positifs des mésappariements extemes de 3 à 4%, qui correspond en fait à un taux de faux positifs d'environ 10% pour 30 et 40% des mésappariements externes. De tels taux de faux positifs et de faux négatifs représentent une amélioration considérable de l'état de l'art procédant par hybridation isotherme. De tels taux sont sufisamment faibles pour permettre la reconstruction de la séquence d'un échantillon nucléique par séquençage par hybridation. En effet, les erreurs d'hybridation restantes peuvent être corrigées par un logiciel d'analyse de données adéquat grâce à la redondance des hybridations (5).

Pour améliorer encore la qualité des résultats, on pourra procéder à deux expériences d'hybridation par échantillon au lieu d'une seule. Le taux de faux positif devrait alors être de l'ordre de 1% pour 30 à 40% des mésappariements externes.

**TABLEAU 1**

| Oligonucléotides | Hybridation |
|---|---|
| bA:fDc | 0,31 |
| bB:fBc | 0,86 |
| bC:fBc | 0,06 |
| bD:fDc | 0,73 |
| bE:fDc | 0,02 |
| bF:fBc | 0,14 |
| bG:fDc | 1,03 |
| bH:fJc | 0,14 |
| bI:fJc | 0,79 |
| bJ:fJc | 1,00 |

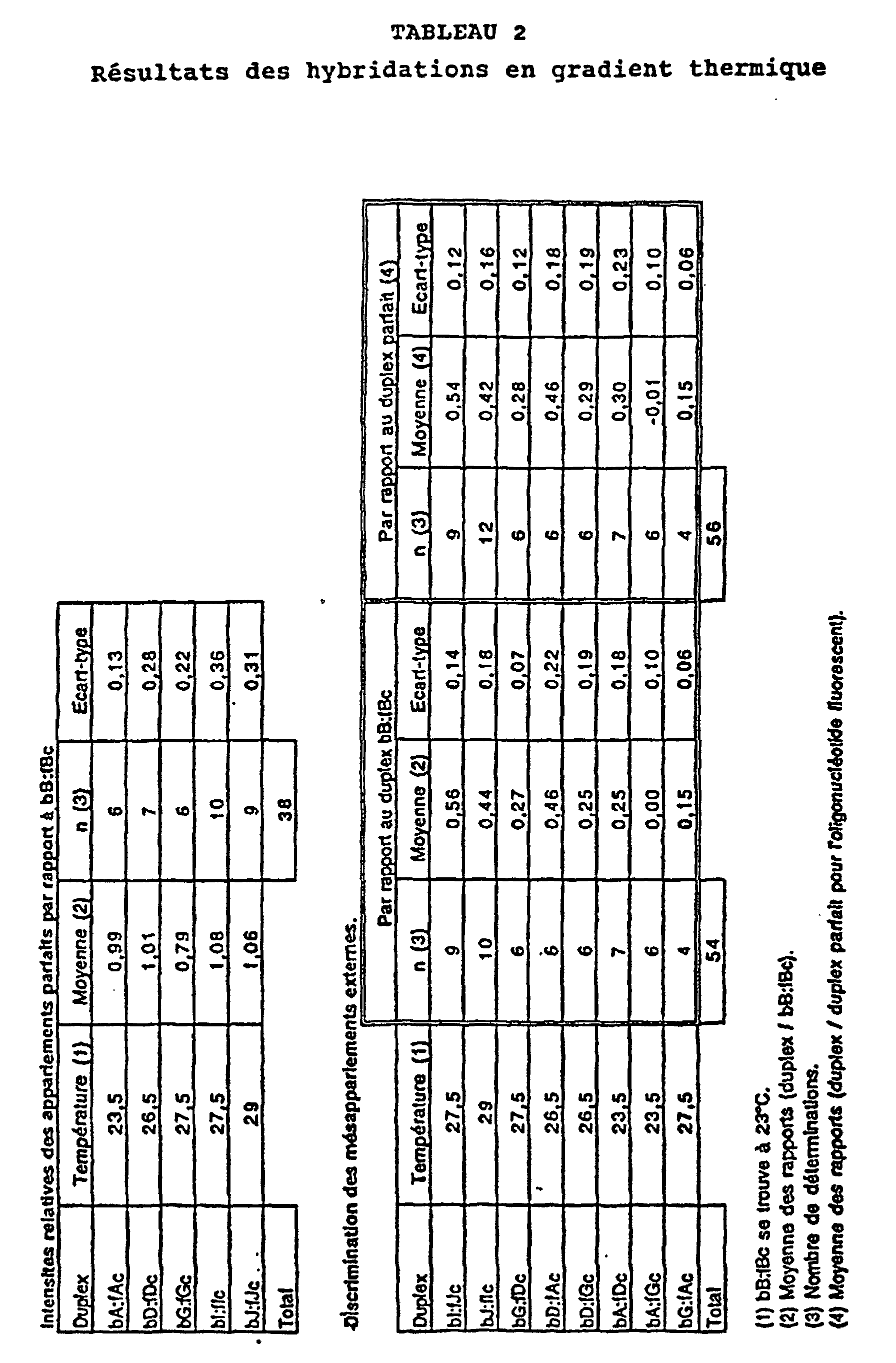

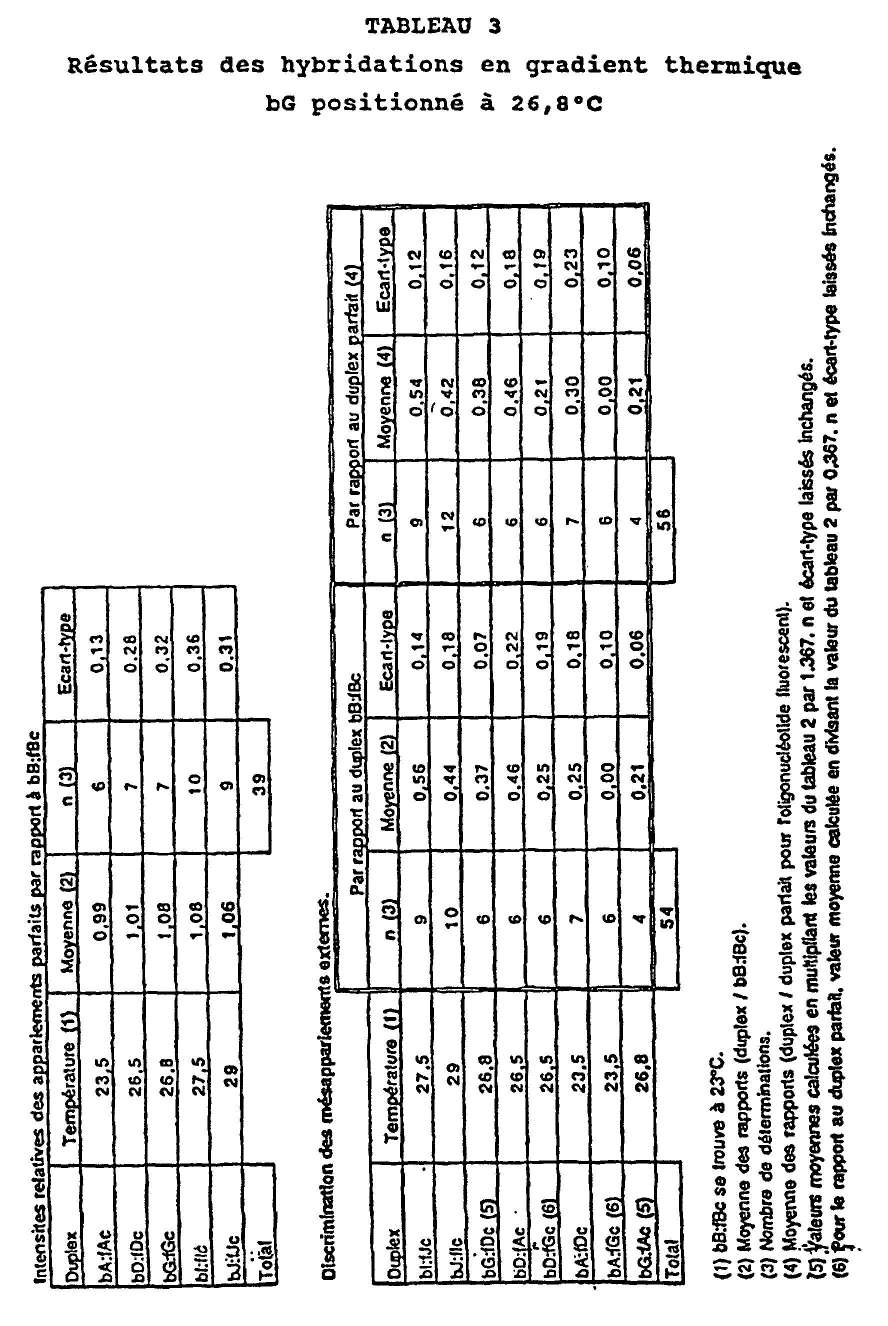

### Références bibliographiques incorporées à la présente description, en tant que de besoin

(1) K. R. Khrapko, Y. P. Lysov, A. A. Khorlyn. V. V. Shick, V. L. Florentiev and A. D. Mirzabekov; An oligonucleotide hybridization approach to DNA sequencing; FEBS Letters; 1989; **256;** 118-122.
(2) M. J. Doktycz, M. D. Morris, S. J. Dormady, K, L. Beattie and K. B. Jacobson; optical melting of 128 octamer DNA duplexes; J. Biol. Chem.; 1995; **270;** 8439-8445.
(3) K. J. Breslauer, R. Frank, H. Blöcker and L. A. Mary; Predicting DNA duplex stabilité from the base sequence; Proc. Natl. Acad. Sci. USA; 1986; 83; 3746-3750.
(4) U. Maskos and E. M. Southern; A study of oligonucleotide reassociation using large arrays of oligonucleotides synthesised on a glass support; Nucleic Acids Research; 1993; **21**; 4663-4669.
(5) Z. Strezoska, T. Paunesku, D. Radosavljevic, 1. Labat, R. Drmanac and R. Crkvenjak-ov; DNA sequencing by hybridization: 100 bases read by a non-gel-based method; Proc. Natl. Acad. Sci. USA; 1991; **88**; 10089-10093.
(6) EM Southern et al (1992); Analysing and comparing nucleic acid sequences by hybridization to arrays of oligonucleotides; evaluation using experimental models. Genomics 13, 1008-1017.
(7) R. Drmanac et al (1993); DNA sequence determination by hybriization : a strategy for efficient large-scale sequencing Science 260; 1649-1652.
(8) Carrano et al (1989); A high resolution, fluorescence based, semi-automated method for DNA fingerprinting, Genomic 4, 129-136.
(9) Lysov, Yu et al (1988); Doklady akademi. Nauk. SSR 303: 1508-1511.
(10) Drmanac, R et al (1989) Genomics 4: 114-128.
(11) Sze VLSI Technology, Mc Graw Hill 1983.

## Revendications

1. Procédé de traitement par complexation d'un milieu liquide comprenant un ou plusieurs composants déterminant la présence dans ledit milieu liquide d'une pluralité de ligands différents à l'état libre, selon lequel :
a) on dispose d'un support de complexation plurale, sur lequel sont distribués une pluralité de sites de couplage, séparés les uns des autres, dans lesquels sont immobilisés une pluralité d'anti-ligands respectivement différents, et susceptibles d'être complexés avec lesdits ligands respectivement,
b) on met en contact ledit milieu liquide avec ledit support de complexation, moyennant quoi lesdits ligands s'apparient avec lesdits anti-ligands respectivement, et sont fixés audit support,
**caractérisé en ce que** la stabilité de chaque complexe ligand/anti-ligand étant dépendante, directement ou indirectement, d'au moins un paramètre exogène, dit de référence, conditionnant ledit support, d'une part on différencie les sites de couplage sur le support, en fonction dudit paramètre exogène de référence, ayant des valeurs respectivement différentes dans lesdits sites, et d'autre part on distribue les anti-ligands entre les différents sites de couplage, en fonction de valeurs optimum prédéterminées du paramètre exogène de référence, déterminant la stabilité des différents complexes ligand/anti-ligand respectivement.

2. Procédé selon la revendication 1, **caractérisé en ce qu'**on effectue la mise en contact selon (b) en appliquant une même valeur minimum du paramètre exogène de référence à tous les sites de couplage, suffisante pour complexer pratiquement tous les ligands avec les anti-ligands, puis on lave le support en appliquant aux différents sites de couplage des valeurs respectivement différentes, et prédéterminées, du paramètre exogène de référence.

3. Procédé selon la revendication 1, **caractérisé en ce qu'**on effectue la mise en contact selon (b) en appliquant aux différents sites de couplage des premières valeurs respectivement différentes, et prédéterminées, du paramètre exogène de référence, puis on lave le support en maintenant les différents sites de couplage à des secondes valeurs prédéterminées du paramètre exogène de référence, respectivement différentes; les premières valeurs étant identiques ou différentes des secondes valeurs respectivement du paramètre exogène de référence.

4. Procédé selon la revendication 1, **caractérisé en ce qu'**on effectue la mise en contact du support avec le milieu liquide, en appliquant aux différents sites de couplage des valeurs respectivement différentes et prédéterminées du paramètre exogène de référence, on lave le support hybridé avec un liquide de lavage à température relativement froide et constante.

5. Procédé selon la revendication 1, **caractérisé en ce qu'**on établit sur le support une gradation spatiale du paramètre exogène de référence, notamment un gradient dudit paramètre, selon au moins une direction de référence dudit support, ladite gradation spatiale fixe la position des différents sites de couplage, et la distribution des anti-ligands entre lesdits sites de couplage respectivement.

6. Procédé selon la revendication 1, **caractérisé en ce que** le paramètre exogène de référence est :
- ou un paramètre ou condition physique, par exemple la température
- ou un paramètre ou condition chimique, biochimique, ou biologique à la surface du support de complexation, par exemple la concentration surfacique d'un anti-anti-ligand susceptible de s'apparier avec pratiquement tous les anti-ligands
- ou un paramètre physico-chimique de la surface du support, par exemple sa mouillabilité ou tension superficielle par rapport au milieu liquide.

7. Procédé selon la revendication 1, **caractérisé en ce qu'**au moins un autre paramètre exogène duquel dépend la stabilité des différents complexes ligand/anti-ligand, est fixé à une valeur moyenne entre celles dudit autre paramètre, requises pour la stabilité des différents complexes ligand/anti-ligand respectivement.

8. Procédé selon les revendications 1, 2, 3 ou 4, **caractérisé en ce que** d'une part on différencie les sites de couplage sur le support, en fonction d'un autre paramètre exogène de référence, ayant des valeurs différentes dans lesdits sites, et d'autre part on distribue les anti-ligands entre les différents sites de couplage, également en fonction des valeurs optimum prédéterminées de l'autre paramètre exogène de référence, déterminant aussi la stabilité des différents complexes ligand/anti-ligand respectivement.

9. Procédé selon l'une des revendications 1 à 4, **caractérisé en ce qu'**on observe aux différents sites de couplage du support une grandeur représentative de la présence et/ou de la quantité des différents complexes ligand/anti-ligand, pour obtenir des signaux et/ou des informations représentatifs de la présence et/ou de la quantité des différents ligands dans le milieu liquide de départ.

10. Procédé selon la revendication 9, **caractérisé en ce qu'**on observe les différents complexes ligand/anti-ligand, en mettant en oeuvre au moins l'une quelconque des opérations suivantes, à savoir marquage du ou des composants du milieu liquide et marquage des différents complexes ligand/anti-ligand fixés sur le support.

11. Procédé selon la revendication 1, **caractérisé en ce que** le milieu liquide est obtenu à partir d'un échantillon, par exemple un échantillon ou un matériel biologique, contenant une ou plusieurs entités différentes, à analyser.

12. Procédé selon la revendication 11, **caractérisé en ce que** l'échantillon est soumis à au moins une opération préalable, choisie pour obtenir un ou plusieurs composants dans le milieu liquide traité, identiques à ou dérivés directement des différentes entités respectivement, et représentatives de ces dernières.

13. Procédé selon la revendication 1, **caractérisé en ce que** le support de complexation sur lequel sont fixés les différents ligands est soumis à au moins une opération postérieure, choisie pour séparer dudit support le ou les composants n'ayant pas été couplés, et/ou éluer ou relarguer les différents ligands, de manière différenciée ou non.

14. Procédé selon la revendication 1, **caractérisé en ce qu'**un ligand est un acide nucléique, qui comprend une séquence nucléotidique cible, notamment sous forme monocaténaire, accessible à une hybridation avec une autre séquence nucléotidique sonde, complémentaire, comprise par un anti-ligand correspondant.

15. Procédé selon la revendication 1, **caractérisé en ce que** le milieu liquide comprend plusieurs composants différents, comprenant des ligands respectivement différents, moyennant quoi lesdits composants différents sont fixés et séparés les uns des autres sur ledit support de complexation.

16. Procédé selon la revendication 15, **caractérisé en ce qu'**il est mis en oeuvre dans au moins l'une quelconque des applications suivantes, à savoir identification, quantification, séparation et fractionnement desdites molécules différentes.

17. Procédé selon la revendication 1, **caractérisé en ce que** le milieu liquide comprend un seul composant, comprenant ou exhibant plusieurs ligands respectivement différents, moyennant quoi le même composant est apparié aux différents anti-ligands respectivement.

18. Procédé selon les revendications 14 et 17, **caractérisé en ce que** le seul composant est un acide nucléique, dont la suite de nucléotides d'au moins un brin détermine une pluralité de régions d'hybridation, et donc de ligands, selon les séquences nucléotidiques cible respectivement complémentaires aux séquences nucléotidiques sonde des anti-ligands.

19. Procédé selon la revendication 18, **caractérisé en ce qu'**il est mis en oeuvre dans au moins l'une quelconque des applications suivantes, à savoir séquençage par hybridation du composant nucléique du milieu liquide reséquençage du composant nucléique, analyse et identification d'un matériel ou échantillon nucléique, typage d'un matériel nucléique.

20. Procédé selon les revendications 11 et 14, **caractérisé en ce que** le milieu liquide est obtenu à partir d'un échantillon nucléique, comprenant un seul et même fragment, ou plusieurs fragments nucléiques différents.

21. Procédé selon la revendication 20, **caractérisé en ce que** l'échantillon nucléique est soumis à au moins une opération préalable, choisie parmi les opérations suivantes, dénaturation, clonage, amplification.

22. Dispositif de traitement par complexation d'un milieu liquide comprenant un ou plusieurs composants déterminant la présence dans ledit milieu liquide d'une pluralité de ligands différents à l'état libre, ledit dispositif comprenant :
- un support de complexation plurale, sur lequel sont distribués une pluralité de sites de couplage, séparés les uns des autres, dans lesquels sont immobilisés une pluralité d'anti-ligands respectivement différents, et susceptibles d'être complexés avec lesdits ligands respectivement,
- un moyen de mise en contact dudit milieu liquide avec ledit support, agencé pour apparier lesdits ligands avec lesdits anti-ligands respectivement, et les fixer audit support
**caractérisé en ce que**, la stabilité de chaque complexe ligand/anti-ligand étant dépendante directement ou indirectement d'au moins un paramètre exogène, dit de référence, conditionnant ledit support, d'une part ledit dispositif comprend en outre des moyens de différenciation des sites de couplage sur le support, en fonction dudit paramètre exogène de référence, avec des valeurs respectivement différentes dudit paramètre dans lesdits sites, et d'autre part les anti-ligands sont distribués sur ledit support, entre les différents sites de couplage, en fonction de valeurs optimum prédéterminées dudit paramètre exogène de référence, déterminant la stabilité des différents complexes ligand/anti-ligand respectivement.

23. Dispositif selon la revendication 22, **caractérisé en ce que**, le paramètre exogène de référence retenu pour la complexation, étant la température, lesdits moyens de différenciation sont agencés pour calibrer la température selon au moins une direction de référence, par exemple selon un gradient de températures.

24. Dispositif selon la revendication 23, **caractérisé en ce qu'**il comprend une source froide et une source chaude, entre lesquelles s'étend un support (2) selon ladite direction de référence.

25. Dispositif selon la revendication 22, **caractérisé en ce qu'**il comprend un ou des capteurs, associés aux différents sites de couplage du support respectivement, détectant chacun au moins une grandeur observée, représentative de la présence et/ou de la quantité du complexe ligand/anti-ligand, en chaque site de couplage, et délivrant un ou des signaux de mesure ou informations correspondantes.

26. Dispositif selon le revendication 25, **caractérisé en ce qu'**il comprend une unité de traitement des signaux délivrés par les différents capteurs, selon des opérations mathématiques et/ou logiques prédéterminées, pour obtenir une ou des informations caractérisant le ou les composants du milieu liquide.

27. Dispositif selon la revendication 26, **caractérisé en ce que** la grandeur observée est choisie parmi les grandeurs suivantes, à savoir l'absorbance lumineuse, l'intensité d'une lumière émise ou réémise, et toute propriété physique, optique, électrique, diélectrique ou électrochimique des différents complexes ligand/anti-ligand.

28. Dispositif selon la revendication 22, **caractérisé en ce que** le support est plan.

29. Dispositif selon la revendication 22, **caractérisé en ce que** le support comprend au moins l'un des matériaux suivants, du verre, un matériau poreux, un matériau vitreux, inorganique, ou amorphe, et une matière plastique.

## Claims

1. Method of treatment by complexing of a liquid medium comprising one or more components determining the presence in said liquid medium of a plurality of different ligands in the free state, according to which:
a) a support for plural complexing, is provided on which there are distributed a plurality of coupling sites, separated from each other, in which there are immobilized a plurality of anti-ligands which are respectively different and capable of being complexed with said ligands respectively,
b) said liquid medium is brought into contact with said complexing support, whereby said ligands pair with said anti-ligands respectively and are attached to said support,
**characterized in that** the stability of each ligand/anti-ligand complex being directly or indirectly dependent on at least one exogenous parameter, called reference parameter, conditioning said support, on the one hand, the coupling sites on the support are differentiated as a function of said exogenous reference parameter having values which are respectively different in said sites, and, on the other hand, the anti-ligands are distributed between the different coupling sites as a function of the predetermined optimum values of the exogenous reference parameter determining the stability of the different ligand/anti-ligand complexes respectively.

2. Method according to Claim 1, **characterized in that** the bringing into contact according to (b) is performed by applying the same minimum value of the exogenous reference parameter to all the coupling sites, sufficient to complex practically all the ligands with the anti-ligands, and then the support is washed by applying to the different coupling sites exogenous reference parameter values which are respectively different and predetermined.

3. Method according to Claim 1, **characterized in that** the bringing into contact according to (b) is carried out by applying to the different coupling sites first exogenous parameter values which are respectively different and predetermined, the support is then washed by maintaining the different coupling sites at second predetermined values of the exogenous reference parameter which are respectively different; the first values being identical or different from the second values respectively of the exogenous reference parameter.

4. Method according to Claim 1, **characterized in that** the support is brought into contact with the liquid medium by applying to the different coupling sites exogenous reference parameter values which are respectively different and predetermined, the hybridized support is washed with a washing liquid at a relatively cold and constant temperature.

5. Method according to Claim 1, **characterized in that** a spatial gradation of the exogenous reference parameter is established on the support, in particular a gradient of said parameter, in at least one reference direction of said support, said spatial gradation fixes the position of the different coupling sites, and the distribution of the anti-ligands between said coupling sites respectively.

6. Method according to Claim 1, **characterized in that** the exogenous reference parameter is:
- either a physical parameter or condition, for example the temperature
- or a chemical, biochemical or biological parameter or condition at the surface of the support, for example the surface concentration of an anti-anti-ligand capable of pairing with practically all the anti-ligands
- or of a physicochemical parameter of the surface of the support, for example its wettability or surface tension relative to the liquid medium.

7. Method according to Claim 1, **characterized in that** at least another exogenous parameter on which the stability of the different ligand/anti-ligand complexes depends is set at a mean value between those of said other parameter which are required for the stability of the different ligand/anti-ligand complexes respectively.

8. Method according to Claims 1, 2, 3 or 4, **characterized in that**, on the one hand, the coupling sites on the supports are differentiated as a function of another exogenous reference parameter, having different values in said sites, and, on the other hand, the anti-ligands are distributed between the different coupling sites, also as a function of predetermined optimum values of the other exogenous reference parameter which also determines the stability of the different ligand/anti-ligand complexes respectively.

9. Method according to one of Claims 1 to 4, **characterized in that** a variable representative of the presence and/or of the quantity of the different ligand/anti-ligand complexes is observed at the different coupling sites of the support in order to obtain signals and/or information which are representative of the presence and/or of the quantity of the different ligands in the original liquid medium.

10. Method according to Claim 9, **characterized in that** the different ligand/anti-ligand complexes are observed using at least any one of the following operations, namely labeling of the component(s) of the liquid medium and labeling of the different ligand/anti-ligand complexes attached to the support.

11. Method according to Claim 1, **characterized in that** the liquid medium is obtained from a sample, for example a biological sample or material containing one or more different entities to be analyzed.

12. Method according to Claim 11, **characterized in that** the sample is subjected to at least one preliminary operation chosen so as to obtain one or more components in the liquid medium treated which are identical to or derived directly from the different entities respectively, and which are representative of the latter.

13. Method according to Claim 1, **characterized in that** the complexing support onto which the different ligands are attached is subjected to at least one subsequent operation, chosen in order to separate from said support the component(s) which have not been coupled, and/or to elute or release the different ligands, differentially or otherwise.

14. Method according to Claim 1, **characterized in that** a ligand is a nucleic acid which comprises a target nucleotide sequence, in particular in single-stranded form, which is accessible to hybridization with another complementary probe nucleotide sequence comprised by a corresponding anti-ligand.

15. Method according to Claim 1, **characterized in that** the liquid medium comprises several different components comprising ligands which are respectively different, whereby said different components are attached and separated from each other on said complexing support.

16. Method according to Claim 15, **characterized in that** it is carried out in at least any one of the following applications, namely identification, quantification, separation and fractionation of said different molecules.

17. Method according to Claim 1, **characterized in that** the liquid medium comprises a single component, comprising or exhibiting several ligands which are respectively different, whereby the same component is paired with the different anti-ligands respectively.

18. Method according to Claims 14 and 17, **characterized in that** the sole component is a nucleic acid in which the succession of nucleotides of at least one strand determines a plurality of hybridization regions, and therefore of ligands, according to the target nucleotide sequences, which are respectively complementary to the probe nucleotide sequences of the anti-ligands.

19. Method according to Claim 18, **characterized in that** it is carried out in at least any one of the following applications, namely sequencing by hybridization of the nucleic component of the liquid medium, resequencing of the nucleic component, analysis and identification of a nucleic material or sample, typing of a nucleic material.

20. Method according to Claims 11 and 14, **characterized in that** the liquid medium is obtained from a nucleic sample comprising one and the same fragment, or several different nucleic fragments.

21. Method according to Claim 20, **characterized in that** the nucleic sample is subjected to at least one preliminary operation chosen from the following operations, denaturation, cloning and amplification.

22. Device for treatment by complexing of a liquid medium comprising one or more components determining the presence, in said liquid medium, of a plurality of different ligands in the free state, said device comprising:
- a plural complexing support on which there are distributed a plurality of coupling sites, separated from each other, in which there are immobilized a plurality of anti-ligands which are respectively different and which are capable of being complexed with said ligands respectively,
- a means for bringing said liquid medium into contact with said support, designed so as to pair said ligands with said anti-ligands respectively and to attach them to said support
**characterized in that** the stability of each ligand/anti-ligand complex being directly or indirectly dependent on at least one exogenous parameter, called reference parameter, conditioning said support, on the one hand, said device comprises, in addition, means for differentiating the coupling sites on the support, as a function of said exogenous reference parameter, with values of said parameter which are respectively different in said sites, and, on the other hand, the anti-ligands are distributed on said support, between the different coupling sites, as a function of the predetermined optimum values of said exogenous reference parameter determining the stability of the different ligand/anti-ligand complexes respectively.

23. Device according to Claim 22, **characterized in that** the exogenous reference parameter selected for complexing being the temperature, said differentiating means are designed to grader [sic] the temperature according to at least one reference direction, for example according to a temperature gradient.

24. Device according to Claim 23, **characterized in that** it comprises a cold source and a hot source, between which extends a substrate (2) in said reference direction.

25. Device according to Claim 22, **characterized in that** it comprises one or more sensors, combined with the different coupling sites of the support respectively, each detecting at least one observed variable representative of the presence and/or of the quantity of the ligand/anti-ligand complex at each coupling site, and delivering one or more measuring signals or corresponding information.

26. Device according to Claim 25, **characterized in that** it comprises a unit for processing the signals delivered by the different sensors based on predetermined mathematical and/or logical operations, so as to obtain information characterizing the component(s) in the liquid medium.

27. Device according to Claim 26, **characterized in that** the observed variable is chosen from the following variables, namely light absorbance, the intensity of an emitted or re-emitted light, and any physical, optical, electrical, dielectric or electrochemical property of the different ligand/anti-ligand complexes.

28. Device according to Claim 22, **characterized in that** the support is flat.

29. Device according to Claim 22, **characterized in that** the support comprises at least one of the following materials, glass, a porous material, a vitreous, inorganic or amorphous material, and a plastic mater

## Patentansprüche

1. Verfahren zur Behandlung eines wässrigen Milieus durch Komplexierung, wobei das wässrige Milieu eine oder mehrere Komponenten enthält, die das Vorhandensein einer Vielzahl von in freiem Zustand befindlichen verschiedenen Liganden in dem wässrigen Milieu bestimmen, bei dem:
a) ein Träger für mehrfache Komplexierung bereitgestellt wird, auf dem eine Vielzahl von voneinander getrennten Kopplungsstellen angeordnet sind, an denen eine Vielzahl von jeweils verschiedenen Anti-Liganden immobilisiert sind, die dazu in der Lage sind, mit den jeweiligen Liganden zu komplexieren,
b) das genannte wässrige Milieu mit dem Träger für die Komplexierung in Kontakt gebracht wird, wodurch sich die genannten Liganden mit den genannten jeweiligen Anti-Liganden paaren und an dem Träger fixiert werden,
**dadurch gekennzeichnet, dass** die Stabilität eines jeden Komplexes aus Ligand und Anti-Ligand direkt oder indirekt von wenigstens einem exogenen, Referenzparameter genannten Parameter abhängt, wodurch der genannte Träger konditioniert wird, einerseits die Kopplungsstellen auf dem Träger in Funktion des exogenen Referenzparameters unterschieden werden, der an den Stellen jeweils verschiedene Werte aufweist, und andererseits die Anti-Liganden auf die verschiedenen Kopplungsstellen in Funktion von Optimalwerten verteilt werden, die durch den exogenen Referenzparameter vorbestimmt werden, wodurch die Stabilität der verschiedenen Komplexe aus jeweils Ligand und Anti-Ligand bestimmt wird.

2. Verfahren nach Anspruch 1, **dadurch gekennzeichnet, dass** der Kontakt gemäß Schritt b) dadurch bewirkt wird, dass an allen Kopplungsstellen derselbe Minimalwert des exogenen Referenzparameters angelegt wird, der ausreicht, um praktisch alle Liganden mit den Anti-Liganden zu komplexieren, und der Träger dann gewaschen wird, wobei an verschiedene Kopplungsstellen jeweils verschiedene und vorbestimmte Werte des exogenen Referenzparameters angelegt werden.

3. Verfahren nach Anspruch 1 **dadurch gekennzeichnet, dass** der Kontakt gemäß Schritt b) dadurch hergestellt wird, dass an verschiedene Kopplungsstellen erste, jeweils verschiedene und vorbestimmte Werte des exogenen Referenzparameters angelegt werden, und dann der Träger gewaschen wird, wobei die verschiedenen Kopplungsstellen auf jeweils verschiedene, vorbestimmte zweite Werte des exogenen Referenzparameters gehalten werden, wobei die ersten Werte jeweils identisch oder verschieden zu den jeweiligen zweiten Werten des exogenen Referenzparameters sind.

4. Verfahren nach Anspruch 1, **dadurch gekennzeichnet, dass** der Kontakt des Trägers mit dem wässrigen Milieu dadurch bewirkt wird, dass an verschiedene Kopplungsstellen jeweils verschiedene und vorherbestimmte Werte des exogenen Referenzparameters angelegt werden, und dass der hybridisierte Träger mit einer Waschflüssigkeit bei einer vergleichsweise kühlen und konstanten Temperatur gewaschen wird.

5. Verfahren nach Anspruch 1, **dadurch gekennzeichnet, dass** auf dem Träger längs wenigstens einer Referenzrichtung des Trägers eine räumliche Abstufung des exogenen Referenzparameters, insbesondere ein Gradient des genannten Parameters, etabliert wird, wobei die genannte räumliche Abstufung die Position der verschiedenen Kopplungsstellen und die Verteilung der Anti-Liganden auf die jeweiligen Kopplungsstellen fixiert.

6. Verfahren nach Anspruch 1, **dadurch gekennzeichnet, dass** der exogene Referenzparameter
- entweder ein physikalischer Parameter oder Zustand, bspw. die Temperatur,
- oder ein chemischer, biochemischer oder biologischer Parameter oder Zustand auf der Oberfläche des Trägers für die Komplexierung, z.B. die Oberflächenkonzentration eines Anti-Anti-Liganden, der dazu in der Lage ist, sich mit praktisch allen Anti-Liganden zu paaren,
- oder ein physiko-chemischer Parameter der Oberfläche des Trägers ist, z.B. ihre Benetzbarkeit oder Oberflächenspannung bezogen auf das wässrige Milieu.

7. Verfahren nach Anspruch 1, **dadurch gekennzeichnet, dass** wenigstens ein anderer exogener Parameter, von dem die Stabilität der verschiedenen Komplexe aus Ligand und Anti-Ligand abhängt, auf einen mittleren innerhalb der Werte des genannten anderen Parameters gesetzt wird, die für die Stabilität der verschiedenen Komplexe aus jeweiligem Ligand und Anti-Ligand erforderlich sind.

8. Verfahren nach den Ansprüchen 1, 2, 3 oder 4, **dadurch gekennzeichnet, dass** einerseits die Kopplungsstellen auf dem Träger in Funktion von einem weiteren exogenen Referenzparameter unterschieden werden, der an den genannten Stellen verschiedene Werte aufweist, und andererseits die Anti-Liganden auf die verschiedenen Kopplungsstellen ebenfalls in Funktion von vorbestimmten Optimalwerten des anderen exogenen Referenzparameters verteilt werden, wodurch gleichfalls die Stabilität der verschiedenen Komplexe aus jeweiligem Ligand und Anti-Ligand bestimmt wird.

9. Verfahren nach einem der Ansprüche 1 bis 4, **dadurch gekennzeichnet, dass** an verschiedenen Kopplungsstellen des Trägers eine Größe aufgenommen wird, die für das Vorhandensein und/oder die Quantität der verschiedenen Komplexe aus Ligand und Anti-Ligand repräsentativ ist, um Signale und/oder Informationen zu erhalten, die repräsentativ für das Vorhandensein und/oder die Quantität der verschiedenen Liganden in dem wässrigen Ausgangsmilieu sind.

10. Verfahren nach Anspruch 9, **dadurch gekennzeichnet, dass** die verschiedenen Komplexe aus Ligand und Anti-Ligand erfasst werden, indem wenigstens eine der folgenden Operationen durchgeführt wird, nämlich Markierung des oder der Komponenten des wässrigen Milieus und Markierung der verschiedenen, auf dem Träger fixierten Komplexe aus Ligand und Anti-Ligand.

11. Verfahren nach Anspruch 1, **dadurch gekennzeichnet, dass** das wässrige Milieu ausgehend von einer Probe, bspw. einer biologischen Probe oder einem biologischen Material erhalten wird, worin eine oder mehrere verschiedene Entitäten enthalten sind, die zu analysieren sind.

12. Verfahren nach Anspruch 11, **dadurch gekennzeichnet, dass** die Probe wenigstens einer vorherigen Behandlung unterzogen wird, die ausgewählt ist, um in dem behandelten wässrigen Milieu eine oder mehrere Komponenten zu erhalten, die identisch zu oder direkt abgeleitet sind von jeweils verschiedenen Entitäten und für letztere repräsentativ sind.

13. Verfahren nach Anspruch 1, **dadurch gekennzeichnet, dass** der Träger für die Komplexierung, auf dem die verschiedenen Liganden fixiert sind, wenigstens einer Folgebehandlung unterzogen wird, die ausgewählt wird, um von dem genannten Träger. die Komponente oder die Komponenten zu trennen, die nicht gekoppelt wurden, und/oder um die verschiedenen Liganden ggf. auf unterschiedliche Weise zu eluieren oder auszusalzen.

14. Verfahren nach Anspruch 1, **dadurch gekennzeichnet, dass** ein Ligand eine Nukleinsäure ist, die eine Nukleotid-Zielsequenz umfasst, insbesondere in einsträngiger Form, die einer Hybridisierung mit einer anderen, komplementären Nukleotid-Sondensequenz zugänglich ist, die in einem entsprechenden Anti-Liganden vorhanden ist.

15. Verfahren nach Anspruch 1, **dadurch gekennzeichnet, dass** das wässrige Milieu mehrere verschiedene Komponenten umfasst, die jeweils verschiedene Liganden umfassen, wodurch die verschiedenen Komponenten auf dem Träger für die Komplexierung fixiert und voneinander getrennt werden.

16. Verfahren nach Anspruch 15, **dadurch gekennzeichnet, dass** es für wenigstens eine den folgenden Anwendungen eingesetzt wird, nämlich Identifizierung, Quantifizierung, Trennung und Fraktionierung der verschiedenen Moleküle.

17. Verfahren nach Anspruch 1, **dadurch gekennzeichnet, dass** das wässrige Milieu eine einzige Komponente umfasst, die mehrere, jeweils verschiedene Liganden umfasst oder aufweist, wodurch dieselbe Komponente mit jeweils verschiedenen Anti-Liganden gepaart wird.

18. Verfahren nach den Ansprüchen 14 und 17, **dadurch gekennzeichnet, dass** die einzige Komponente eine Nukleinsäure ist, deren Nukleotidabfolge in wenigstens einem Abschnitt eine Vielzahl von Hybridisierungsregionen, und folglich von Liganden, in Übereinstimmung mit den Nukleotid-Zielsequenzen bestimmt, die jeweils komplementär zu den Nukleotid-Sondensequenzen der Anti-Liganden sind.

19. Verfahren nach Anspruch 18, **dadurch gekennzeichnet, dass** es für wenigstens eine der folgenden Anwendungen eingesetzt wird, nämlich Sequenzierung durch Hybridisierung der Nukleinsäurekomponente des wässrigen Milieus, Resequenzierung der Nukleinsäurekomponente, Analyse und Identifizierung eines Nukleinsäure-Materials oder einer Nukleinsäure-Probe, Typisierung eines Nukleinsäure-Materials.

20. Verfahren nach den Ansprüchen 11 und 14, **dadurch gekennzeichnet, dass** das wässrige Milieu ausgehend von einer Nukleinsäure-Probe erhalten wird, die ein einziges Fragment oder mehrere, verschiedene Nukleinsäure-Fragmente enthält.

21. Verfahren nach Anspruch 20, **dadurch gekennzeichnet, dass** die Nukleinsäure-Probe wenigstens einer vorherigen Behandlung unterzogen wird, die aus den folgenden Behandlungen ausgewählt ist, nämlich Denaturierung, Klonierung, Amplifizierung.

22. Vorrichtung zur Behandlung eines wässrigen Milieus durch Komplexierung, wobei das wässrige Milieu eine oder mehrere Komponenten enthält, die das Vorhandensein einer Vielzahl von im freien Zustand befindlichen, verschiedenen Liganden in dem wässrigen Milieu bestimmen, wobei die Vorrichtung umfasst:
- einen Träger für mehrfache Komplexierung, auf dem eine Vielzahl von voneinander getrennten Kopplungsstellen angeordnet sind, an denen eine Vielzahl von jeweils verschiedenen Anti-Liganden immobilisiert sind, die dazu geeignet sind, mit den genannten Liganden jeweils zu komplexieren,
- ein Mittel zum Kontaktieren des wässrigen Milieus mit dem genannten Träger, das dazu angeordnet ist, um die genannten Liganden jeweils mit den genannten Anti-Liganden zu paaren und sie auf dem genannten Träger zu fixieren,
**dadurch gekennzeichnet, dass** die Stabilität eines jeden Komplexes aus Ligand und Anti-Ligand direkt oder indirekt von wenigstens einem Referenzparameter genannten exogenen Parameter abhängt, wodurch der genannte Träger konditioniert wird, wobei die Vorrichtung einerseits unter anderem Mittel umfasst, um die Kopplungsstellen auf dem Träger in Funktion des exogenen Referenzparameters mit jeweils verschiedenen Werten des Parameters an den genannten Stellen zu differenzieren, und andererseits die Anti-Liganden auf dem Träger auf die verschiedenen Kopplungsstellen in Funktion von vorbestimmten Optimalwerten des exogenen Referenzparameters verteilt sind, wodurch die Stabilität der verschiedenen Komplexe aus jeweiligem Ligand und Anti-Ligand bestimmt wird.

23. Vorrichtung nach Anspruch 22, **dadurch gekennzeichnet, dass** der für die Komplexierung reservierte exogene Referenzparameter die Temperatur ist, wobei die Mittel für die Differenzierung angeordnet sind, um die Temperatur längs wenigstens einer Referenzrichtung zu kalibrieren, z.B. längs eines Temperaturgradienten.

24. Vorrichtung nach Anspruch 23, **dadurch gekennzeichnet, dass** sie eine Kältequelle und eine Wärmequelle umfasst, zwischen denen sich ein Träger längs der genannten Referenzrichtung erstreckt.

25. Vorrichtung nach Anspruch 22, **dadurch gekennzeichnet, dass** sie einen oder mehrere Sensoren umfasst, die jeweils verschiedenen Kopplungsstellen des Trägers zugeordnet sind und jede wenigstens eine beobachtete Größe erfasst, die repräsentativ für das Vorhandensein und/oder die Quantität des Komplexes aus Ligand und Anti-Ligand an jeder Kopplungsseite sind sowie ein oder mehrere Messsignale oder entsprechende Informationen liefern.

26. Vorrichtung nach Anspruch 25, **dadurch gekennzeichnet, dass** sie eine Einheit zur Bearbeitung von von den verschiedenen Sensoren gelieferten Signalen gemäß vorbestimmter mathematischer und/oder logischer Operationen umfasst, um eine oder mehrere Informationen zu erhalten, die für die Komponente oder die Komponenten des wässrigen Milieus kennzeichnend sind.

27. Vorrichtung nach Anspruch 26, **dadurch gekennzeichnet, dass** die aufgenommene Größe ausgewählt ist aus den folgenden Größen, nämlich Lichtabsorption, Intensität von emittiertem oder re-emittiertem Licht sowie jegliche physikalische, optische, elektrische, dielektrische oder elektrochemische Eigenschaft der verschiedenen Komplexe aus Ligand und Anti-Ligand.

28. Vorrichtung nach Anspruch 22, **dadurch gekennzeichnet, dass** der Träger eben ist.

29. Vorrichtung nach Anspruch 22, **dadurch gekennzeichnet, dass** der Träger wenigstens eines der folgenden Materialien umfasst, nämlich Glas, poröses Material, glasartiges Material, organisch oder amorph, und Plastikmaterial.
